## Europäisches Patentamt

(19) **European Patent Office**

**Office européen des brevets**

(11) Publication number: **0 093 610**

**A2**

(12) # EUROPEAN PATENT APPLICATION

(21) Application number: **83302477.1**

(22) Date of filing: **03.05.83**

(51) Int. Cl.³: **C 07 C 149/42**
C 07 C 149/26, C 07 C 149/06
C 07 C 119/00
//C07C149/437, C07C157/09,
C07D207/335, C07D209/14,
C07D213/40, C07D307/52,
C07D317/58, C07D333/20,
A01N47/28, A01N47/30

(30) Priority: **03.05.82 US 374119**

(43) Date of publication of application:
**09.11.83 Bulletin 83/45**

(84) Designated Contracting States:
**AT BE CH DE FR GB IT LI LU NL SE**

(71) Applicant: **UNIROYAL, INC.**
**1230 Avenue of the Americas Rockefeller Center**
**New York, New York 10020(US)**

(72) Inventor: **McGuinness, James Anthony**
**38 Highland Avenue**
**Naugatuck Connecticut 06770(US)**

(72) Inventor: **Bell, Allyn Roy**
**686 Wiese Road**
**Cheshire New Haven Connecticut 06410(US)**

(74) Representative: **Harrison, Michael Robert et al,**
**URQUHART-DYKES & LORD 11th Floor Tower House**
**Merrion Way**
**Leeds LS2 8PB West Yorkshire(GB)**

(54) **Substituted urea herbicides.**

(57) A compound having the formula R²SCH(R)N(R¹)C(X)-NHR³, useful in the control of weeds is disclosed, the groups R¹, R², R³, and X being as indicated in Tables A,B,C,D,E,F and G of the specification.

Intermediates, methods and reaction mixtures for making the compounds are disclosed.

Also disclosed are herbicidal compositions containing the compounds and methods of controlling weeds using the compounds.

5677

## SUBSTITUTED UREA HERBICIDES

This invention is concerned with substituted urea compounds having the structural formula $R^2SCH(R)N(R^1)C(X)NHR^3$, wherein R, $R^1$, $R^2$, $R^3$ and X are more clearly defined below. These chemicals have excellent pre- and/or postemergence herbicidal activity.

Substituted ureas as well as their herbicidal activity are generally known and are disclosed in the following references of interest:

USP 3,072,719, Jan. 8, 1963 (Beaver et al) teaches bacteriostat chemicals wherein both nitrogen atoms of the urea moiety carry a hydrogen atom. The process by which these chemicals are made does not lead to the compounds of this invention.

USP 3,990,883, Nov. 9, 1976 (Clapot et al) deals with substituted ureas, useful as plant growth regulants, wherein the substituents are distinct and different from those of this invention, especially as far as the $R^1$ and $SR^2$ groups of th.s case are concerned. The process disclosed by this reference cannot produce the instant chemicals.

USP 3,978,123, Aug. 31, 1976 (Chan) disclosed herbicidally active substituted ureas wherein the sulfur atom is separated from one of the nitrogen atoms of the urea moiety by a $CH_2$ group, i.e., a carbon atom having no other substitutents but hydrogen. The compounds of this invention cannot be made by the process taught.

USP 4,043,796, Aug. 23, 1977 (Hainant et al) teaches substituted ureas wherein a thio, sulfinyl or sulfonyl group is connected to one of the urea nitrogen atoms by a non-substituted $-CH_2-$ or $-CH_2CH_2-$ group. The compounds of this case cannot be made by the process described.

USP 4,045,209, Aug. 30, 1977 (Hainant et al) describes compounds similar to USP 4,043,796 and is non-applicable to this invention for the same reasons.

USP 4,111,682, Sept. 5, 1978 (Gutman) teaches substituted ureas wherein an RS group (R being for instance methyl) is attached to one of the urea nitrogen atoms through a $-CH_2-$ link-

age, said linkage carrying no other substituent but hydrogen, whereas in the instant invention said linking moiety must have attached thereto one radical other than hydrogen. The process for obtaining such compounds cannot be used for making the instant compounds.

USP 4,090,864, May 29, 1978 (Gutman) deals with herbicidally active substituted ureas, wherein the equivalent to R of this invention is hydrogen, whereas R in the instant case must be other than hydrogen. The process of this reference does not lead to the chemicals claimed in this application.

USP 4,058,392, Nov. 15, 1977 (Thomas et al) disclosed herbicides having the formula $RN(CH_2XR^1)CON(R^2)R^3$ wherein X may be, among other things, sulfur. These compounds are distinct and different from those claimed herein by the fact that X is linked with one of the urea nitrogen atoms by a $-CH_2-$ group, whereas the instant compounds require an -CHR- moiety, R being other than hydrogen. Furthermore, the equivalent to either $R^2$ or $R^3$ of the reference must be hydrogen in this invention. The instant chemicals cannot be prepared using the process of this publication.

USP 3,916,010, Oct. 28, 1975; USP 3,903,154, Sept. 2, 1975; USP 4,007,033, Feb. 8, 1977; USP 4,012,225, May 15, 1977; USP 4,111,683; Sept. 5, 1978; and USP 4,200,450, April 29, 1980 (all of Singer) disclose substituted urea herbicides wherein the group equivalent to R of this invention must be a polyhalo-substituted methyl group.

USP 3,947,971 Nov. 12, 1974 (Koenig) reveals certain tetrasubstituted ureas, one of the substitutents possibly being a hydrocarbylthio moiety. The instant invention does not contemplate tetrasubstitution, nor could the instant chemicals be made according to the reference process.

Swiss Patent No. 430,323, Aug. 15, 1967 (Schuler) Chem. Abstracts 68, 68660 z (1968) deals with phenyl urea herbicides containing a $-CH_2CH_2SR$ moiety attached to one of the urea nitrogen atoms. The disclosed process would not provide the compounds of this invention.

It is the object of this invention to disclose new and unique substituted urea compounds having herbicidal activity.

It is a further object to provide a process for making the substituted ureas of this invention.

It is still a further object of this invention to provide certain compounds as intermediates for making such substituted urea chemicals.

It is also an object of this invention to teach a method for controlling weeds using the substituted ureas disclosed herein.

Another object of this invention resides with an intermediate reaction mixture useful for making substituted urea chemicals.

The compounds of this invention have the structural formula $R^2SC(H)(R)N(R^1)C(X)NHR^3$ wherein the meanings for R, $R^1$, $R^2$, $R^3$ and X are outlined in Tables A, B and C for compounds of Groups A, B and C, respectively.

Preferably, the radicals of Groups A, B and C have the meanings indicated in Tables D, E and F. Especially good herbicidal activity is obtained from the chemicals listed in Table G.

## Table A

Structural Formula: $R^2SCH(P)N(R^1)C(X)NHR^3$

### Group "A" Type Compounds

| R | R^1 | R^2 | R^3 | X |
|---|---|---|---|---|
| $C_1-C_{11}$ alkyl | $C_1-C_6$ alkyl | $C_1-C_{18}$ alkyl | $C_3-C_6$ cycloalkyl | oxygen |
| $C_3-C_{10}$ alkenyl | $C_3-C_6$ alkenyl | $C_3-C_6$ alkenyl | $C_6-C_{10}$ aryl | sulfur |
| $C_3-C_8$ cycloalkyl | $C_3-C_6$ alkynyl | $C_3-C_8$ cycloalkyl | phenyl having 1-3 substituents, i.e., | |
| $C_3-C_8$ cycloalkenyl | $C_3-C_6$ cycloalkyl | $C_7-C_9$ phenylalkyl | halogen | |
| $C_7-C_9$ phenylalkyl | $C_7-C_9$ phenylalkyl | furanylmethyl | $C_1-C_4$ alkyl | |
| $C_2-C_{10}$ alkoxy- or alkylthioalkyl | | $C_3-C_{21}$ alkoxycarbonylalkyl | trihalomethyl | |
| $C_7-C_{10}$ bridged cycloalkyl or cycloalkenyl | | $C_6-C_{10}$ aryl | $C_1-C_4$ alkoxy or alkylthio | |
| $C_5-C_7$ heterocycloalkyl | | phenyl having 1-2 substituents, i.e. | phenoxy | |
| | | hydroxy | $NO_2$ | |
| | | halogen | CN | |
| | | $C_1-C_4$ alkyl. | | |
| | | $C_1-C_4$ alkoxy | | |

$R^2$ is $-C_nH_{2n}R^4$   n is an integer 2-8

$R^4$ is hydroxy

   $C_1-C_6$ alkoxy, or

   $-NR^5R^6$

   $R^5$ and $R^6$ are the same or different

   and are $C_1-C_8$ alkyl or

   $R^5$ plus $R^6$ is

   $C_4-C_5$ alkylene

   $C_4-C_6$ oxydialkylene, or

$R^4$ is $-OOCNHR^7$

   $R^7$ is $C_1-C_8$ alkyl

      $C_5-C_6$ cycloalkyl

Proviso: If R is cycloalkyl and $R^1$ is alkyl other than methyl, then $R^2$ is not hydroxyphenyl; and if $R^3$ is cycloalkyl, then X is oxygen,

and if R, $R^1$ or $R^2$ are alkenyl, or $R^1$ is alkynyl the unsaturation is not in the 1- position of such radicals.

-4-

Structural Formula: $R^2SCH(R)N(R^1)C(X)NHR^3$

## Group "B" Type Compounds

| R | $R^1$ | $R^2$ | $R^3$ | X |
|---|---|---|---|---|
| $C_6$-$C_{10}$ aryl | $C_1$-$C_4$ alkyl | $C_1$-$C_{12}$ alkyl | phenyl | oxygen |
| furanyl | $C_3$-$C_6$ alkenyl | $C_3$-$C_6$ alkenyl | phenyl having 1-3 | sulfur |
| indolyl | $C_3$-$C_6$ alkynyl | $C_3$-$C_6$ cycloalkyl | substitutents, i.e., | |
| pyridinyl | $C_3$-$C_6$ cycloalkyl | $C_7$-$C_9$ phenylalkyl | fluorine, one in the 2- position | |
| pyrrolyl | benzyl | $C_8$-$C_{10}$ methoxyphenylalkyl | $C_1$-$C_3$ alkyl, one in the 4- position | |
| quinolinyl | | furanylmethyl | $C_1$-$C_3$ alkylthio, one in the 4- position | |
| thienyl | | $C_3$-$C_{21}$ alkoxycarbonylalkyl | provided at least one ortho-hydrogen | |

R = phenyl having 1-3 substituents, i.e.,
hydroxy
halogen
$C_1$-$C_4$ alkyl
$C_1$-$C_8$ alkoxy
phenoxy
methylenedioxy
$C_2$-$C_4$ dialkylamino

$R^2$ = $-C_nH_{2n}R^4$; n is an integer 2-8
$R^4$ is hydroxy
$C_1$-$C_6$ alkoxy
$-NR^5R^6$, $R^5$ and $R^6$ are the same or different and are
$C_1$-$C_8$ alkyl
$R^5$ plus $R^6$ is
$C_4$-$C_5$ alkylene
$C_4$-$C_6$ oxydialkylene
$R^4$ is $-OOCNHR^7$
$R^7$ is $C_1$-$C_8$ alkyl
$C_5$-$C_6$ cycloalkyl.

**Proviso:** If $R^1$ or $R^2$ is alkenyl, or $R^1$ is alkynyl, the unsaturation is not in the 1- position of such radicals.

## Table C

### Structural Formula: $R^2SCH(R)N(R^1)C(X)NHR^3$

### Group "C" Type Compounds

| R | $R^1$ | $R^2$ | $R^3$ | X |
|---|---|---|---|---|
| $C_6$-$C_{10}$ aryl | methyl | phenyl having 1-2 substituents, ie., | phenyl | oxygen |
| furanyl | $C_3$-$C_6$ alkenyl | hydroxy | 3-trifluoromethyl-phenyl | |
| 4-pyridyl | $C_3$-$C_6$ alkynyl | methyl | 2-fluorophenyl | |
| thienyl | | methoxy | 2,3-difluorophenyl | |
| phenyl having 1-3 substituents, i.e., | | | 2,4-difluorophenyl | |
| hydroxy | | | 2,5-difluorophenyl | |
| $C_1$-$C_4$ alkyl | | | 2,6-difluorophenyl | |
| $C_1$-$C_8$ alkoxy | | | 2-fluoro-3-trifluoromethylphenyl | |
| methylenedioxy | | | | |
| $C_2$-$C_4$ dialkylamino | | | | |

Proviso: If $R^1$ is alkenyl or alkynyl, the unsaturation is not in the 1- position of such radicals.

-6-

## Table D
### Compound Structure $R^2SCH(R)N(R^1)C(X)NHR^3$

### Preferred Group "A" Compounds

| R | $R^1$ | $R^2$ | $R^3$ | X |
|---|---|---|---|---|
| $C_1-C_{11}$ alkyl | $C_1-C_4$ alkyl | $C_1-C_{12}$ alkyl | phenyl | Oxygen |
| prop-2-enyl | $C_3-C_4$ alkenyl | $C_3-C_4$ alkenyl | 2-fluorophenyl | |
| cyclopropyl | prop-2-ynyl | $C_7-C_8$ phenylalkyl | 3-fluorophenyl | |
| $C_6-C_7$ cycloalkenyl | cyclopropyl | furfuryl | 4-fluorophenyl | |
| benzyl | benzyl | $C_3-C_{15}$ alkoxycarbonylalkyl | 2,4-difluorophenyl | |
| $C_2-C_{10}$ alkoxy - or | | phenyl | 2,5-difluorophenyl | |
|     alkylthioalkyl | | phenyl having 1-2 substitutents, i.e., | 3,4-dichlorophenyl, and | |
| $C_7-C_8$ bridged cycloalkyl or | |     hydroxy | 4- isopropylphenyl | |
|     cycloalkenyl | |     chlorine | | |
| | |     methyl | | |
| | |     methoxy | | |

Proviso: As in Table A

$R^2$ is $-C_nH_{2n}R^4$,    n is integer 2-4

    $R^4$ is hydroxy

        $C_1-C_2$ alkoxy

        $-NR^5R^6$, $R^5$ and $R^6$

        are the same or different

        and are $C_1-C_2$ alkyl, or

        $R^5$ plus $R^6$ is $C_4-C_5$ alkylene

            $C_4-C_6$ oxydialkylene

    $R^4$ is - $OOCHNR^7$

        $R^7$ is $C_1-C_2$ alkyl

           cyclohexyl

Table L

Compound Structure: $R^2SCH(R)N(R^1)C(X)NHR^3$

Preferred Group "B" Compounds

| R | $R^1$ | $R^2$ | $R^3$ | X |
|---|---|---|---|---|
| phenyl | $C_1$-$C_4$ alkyl | $C_1$-$C_8$ alkyl | phenyl | Oxygen |
| furanyl | $C_3$-$C_4$ alkenyl | $C_3$-$C_4$ alkenyl | 2 fluorophenyl | |
| indolyl | prop-2-ynyl | $C_3$-$C_5$ cycloalkyl | 2,4-difluorophenyl | |
| pyridinyl | | $C_7$-$C_8$ phenylalkyl | 2,5-difluorophenyl | |
| pyrrolyl | | furfuryl | 4-methylthiophenyl | |
| quinolinyl | | $C_3$-$C_{15}$ alkoxycarbonylalkyl | 4-isopropylphenyl | |
| thienyl | | $-C_nH_{2n}R^4$, n is an integer 2-4 | | |
| phenyl having 1-2 | | $R^4$ is hydroxy | | |
| substitutents, i.e., | | $C_1$-$C_4$ alkoxy | | |
| methyl | | $-NR^5R^6$, $R^5$ and $R^6$ | | |
| $C_1$-$C_5$ alkoxy | | are the same or | | |
| methylenedioxy | | different and are | | |
| $C_2$-$C_4$ dialkylamino | | $C_1$-$C_4$ alkyl; | | |
| | | $R^5$ plus $R^6$ is | | |
| | | $C_4$-$C_5$ alkylene | | |
| | | $C_4$-$C_6$ oxydialkylene | | |
| | | $R^4$ is $-OOCNR^7$ | | |
| | | $R^7$ is $C_1$-$C_2$ alkyl | | |

-8-

0093610

## Table F

Compound Structure: $R^2SCH(R)N(R^1)C(X)NHR^3$

### Preferred Group "C" Compounds

| R | $R^1$ | $R^2$ | $R^3$ | X |
|---|---|---|---|---|
| phenyl | methyl | phenyl having 1-2 | 3-trifluoromethylphenyl | oxygen |
| 2-furanyl | $C_3-C_4$ alkenyl | substituents, i.e., | 2-fluorophenyl | |
| 2-thienyl | prop-2-ynyl | hydroxy | 2,4-difluorophenyl | |
| phenyl having 1-2 | | methoxy | 2,5-difluorophenyl | |
| substituents, i.e., | | | | |
| hydroxy | | | | |
| $C_1-C_5$ alkoxy | | | | |

## TABLE G

### Structural Formula: $R^2SCH(R)N(R^1)C(O)NHR^3$

| R | $R^1$ | $R^2$ | $R^3$ |
|---|---|---|---|
| | | **Group "A" Compounds** | |
| $CH(CH_3)_2$ | $CH_3$ | $CH_3$ | $C_6H_5$ |
| $CH_3$ | " | " | $2\text{-}F\text{-}C_6H_4$ |
| $CH(CH_3)_2$ | " | " | " |
| " | " | $n\text{-}C_{12}H_{25}$ | " |
| " | " | $CH_2CH_2OH$ | " |
| " | " | $CH_2CH_2COOCH_3$ | " |
| " | " | $CH_3$ | $2,5\text{-}F_2\text{-}C_6H_3$ |
| $bcC_7H_9$ (1) | " | " | $2\text{-}F\text{-}C_6H_4$ |
| | | **Group "B" Compounds** | |
| $4\text{-}(CH_3O)\text{-}C_6H_4$ | $CH_3$ | $CH_3$ | $2\text{-}F\text{-}C_6H_4$ |
| " | " | $CH_2CH_2COOCH_3$ | " |
| " | " | $CH_2CH_2COOC_8H_{17}$ | " |
| $3,4\text{-}(CH_3O)_2\text{-}C_6H_3$ | " | $CH_3$ | " |
| " | " | $CH_2CH_2COOCH_3$ | " |
| " | " | $CH_3$ | $4\text{-}i\text{-}C_3H_7\text{-}C_6H_4$ |
| $2\text{-furyl}$ | " | " | $2\text{-}F\text{-}C_6H_4$ |
| $3,4\text{-}(CH_3O)_2\text{-}C_6H_3$ | $n\text{-}C_4H_9$ | " | " |
| | | **Group "C" Compounds** | |
| $C_6H_5$ | $CH_3$ | $4\text{-}(CH_3O)\text{-}C_6H_4$ | $2\text{-}F\text{-}C_6H_4$ |
| $2\text{-furyl}$ | " | $4\text{-}C_6H_4OH$ | " |
| $2\text{-thienyl}$ | " | " | " |

Remarks: (1) bicyclo[2.2.1]hept-5-en-2-yl

Within the contemplation of this invention, substituted urea compounds may be prepared having the structural formula $R^2SCH(R)N(R^1)C(X)NHR^3$ wherein R is a saturated or unsaturated aliphatic, cycloaliphatic or aromatic hydrocarbyl group having from 1 to 30 carbon atoms, usually from 1 to 18 carbon atoms, or such a hydrocarbyl group substituted with halogen, hydroxy, $C_1-C_8$ alkoxy, $C_1-C_8$ alkylthio, $C_6-C_{10}$ aryloxy, $C_5-C_7$ heterocycloalkyl, $C_2-C_{12}$ alkoxycarbonyl, methylenedioxy, cyano or nitro, or R is a 5-6 membered heterocyclic moiety containing one or more N, S or O atoms; $R^1$ is an aliphatic or cycloaliphatic, saturated or unsaturated $C_1-C_{18}$ hydrocarbyl group; $R^2$ may be the same as or different from R; and $R^3$ is an aliphatic, cycloaliphatic or aromatic, saturated or unsaturated hydrocarbyl group having 1 to 18 carbon atoms or such a hydrocarbyl group substituted with halogen, trihalomethyl, $C_1-C_8$ alkyl, $C_1-C_8$ alkoxy, $C_1-C_8$ alkylthio, phenoxy, nitro or cyano; and X is oxygen or sulfur; comprising reacting an adduct having the structural formula $RCH(SR^2)NHR^1$ with an iso(thio)-cyanate $R^3NCX$, wherein R, $R^1$, $R^2$, $R^3$ and X have the meanings above, in the presence of an inert, aprotic solvent. Alternatively, the above mentioned substituted urea compounds can be made in situ by reacting the iso(thio)cyanate $R^3NCX$ with a reaction mixture comprising a Schiff base $RCH=NR^1$, a thiol $R^2SH$ and the adduct $RCH(SR^2)NHR^1$ in the presence of an aprotic solvent, inert to the reactants. Either reaction may be conducted at a temperature ranging from -10°C to about 75°C, essentially in the absence of water over a period of 1 to 24 hours.

As a further embodiment, this invention concerns a process for making compounds having the general formula $R^2SCH(R)N(R^1)C-(X)NHR^3$ wherein (A) R is $C_1-C_{11}$ alkyl, $C_3-C_{10}$ alkenyl, $C_3-C_8$ cycloalkyl, $C_5-C_8$ cycloalkenyl, $C_7-C_{10}$ bridged cycloalkyl or cyclo-alkenyl, $C_2-C_{10}$ alkoxyalkyl or alkylthioalkyl, $C_5-C_7$ heterocyclo-alkyl, $C_7-C_9$ phenylalkyl; $R_2$ is $C_1-C_{18}$ alkyl, $C_3-C_6$ alkenyl, $C_3-C_8$ cycloalkyl, $C_7-C_9$ phenylalkyl, furanylmethyl, $C_3-C_{21}$ alkoxycarbonylalkyl, $C_6-C_{10}$ aryl, phenyl substituted with one to two groups, these groups being hydroxy, halogen, $C_1-C_4$ alkyl, $C_1-C_4$ alkoxy or a $-C_nH_{2n}R^4$ group wherein n is an integer from 2 to 8, $R^4$ is hydroxy, $C_1-C_6$ alkoxy, $-NR^5R^6$, wherein $R^5$ and $R^6$

may be the same or different and are $C_1$-$C_8$ alkyl or $R^5$ and $R^6$ together are $C_4$-$C_5$ alkylene or $C_4$-$C_6$ oxydialkylene, or $R^4$ is -OOCNHR$^7$, wherein $R^7$ is $C_1$-$C_8$ alkyl or $C_5$-$C_6$ cycloalkyl; $R^3$ is $C_3$-$C_6$ cycloalkyl, $C_6$-$C_{10}$ aryl, phenyl substituted with one to three groups, said groups being halogen, $C_1$-$C_4$ alkyl, trihalomethyl, $C_1$-$C_4$ alkoxy or alkylthio, phenoxy, nitro or cyano; X is oxygen or sulfur; provided if R is cycloalkyl and $R^1$ is alkyl other than methyl, then $R^2$ is not hydroxyphenyl, and with the further proviso that if $R^3$ is cyclohexyl, then X is oxygen; and if R, $R^1$ or $R^2$ are alkenyl or $R^1$ is alkynyl, the unsaturation is not in the 1-position of such radicals; or wherein (B) R is $C_6$-$C_{10}$ aryl, furanyl, indolyl, pyridinyl, pyrrolinyl, quinolinyl, thienyl or phenyl carrying one to three groups, said groups being hydroxy, halogen, $C_1$-$C_4$ alkyl, $C_1$-$C_8$ alkoxy, phenoxy, methylenedioxy or $C_2$-$C_4$ dialkylamino; $R^1$ is $C_1$-$C_4$ alkyl, $C_3$-$C_6$ alkenyl, $C_3$-$C_6$ alkynyl, $C_3$-$C_6$ cycloalkyl or benzyl; $R^2$ is $C_1$-$C_{12}$ alkyl, $C_3$-$C_6$ alkenyl, $C_3$-$C_6$ cycloalkyl, $C_7$-$C_9$ phenylalkyl, $C_8$-$C_{10}$ methoxyphenylalkyl, furanylmethyl, $C_3$-$C_{21}$ alkoxycarbonylalkyl or $R^5$ and $R^6$ together are $C_4$-$C_5$ alkylene or $C_4$-$C_6$ oxydialkylene, or $R^4$ is -OOCNHR$^7$ wherein $R^7$ is $C_1$-$C_8$ alkyl or $C_5$-$C_6$ cycloalkyl, $R^3$ is phenyl having 1-3 substituents, the substitutents being fluorine, $C_1$-$C_3$ alkyl or $C_1$-$C_3$ alkylthio, at least one of the substituents being either fluorine in the 2- position, or $C_1$-$C_3$ alkyl or $C_1$-$C_3$ alkylthio in the 4-position; provided at least one ortho-hydrogen is present; and X is oxygen or sulfur; or (C) wherein R is $C_6$-$C_{10}$ aryl, furanyl, 4-pyridinyl, thienyl or phenyl substituted with one to three groups, said groups being hydroxy, $C_1$-$C_4$ alkyl, $C_1$-$C_8$ alkoxy, methylenedioxy or $C_2$-$C_4$ dialkylamino; $R^1$ is methyl, $C_3$-$C_6$ alkenyl, $C_3$-$C_6$ alkynyl, $R^2$ is phenyl having one to two groups, said groups being hydroxy, methyl or methoxy; $R^3$ is monofluorophenyl or difluorophenyl, at least one of the fluorine atoms being in the ortho-position; or 3-trifluoromethylphenyl; and X is oxygen; comprising reacting a compound having the structural formula $R^3$NCX, wherein $R^3$ and X have the meanings above, with a compound having the structural formula RCH(SR$^2$)NHR$^1$, wherein R, $R^1$ and $R^2$ have the meanings defined above, or by reacting the compound having the structural formula $R^3$NCX with a reaction mixture comprising the chemicals, having the structural formula $RCH=NR^1$, $R^2SH$ and $RCH(SR^2)NHR^1$, wherein R, $R^1$, $R^2$, $R^3$ and X have the above meanings, in an inert aprotic solvent.

The reaction mixture is obtained by contacting the Schiff base, namely $RCH=NR^1$ with the thiol $R^2SH$ in a suitable solvent, in order to form the adduct $RCH(SR^2)NHR^3$ whereby the reaction mixture encompasses all three species in equilibrium. Schiff bases as well as processes for making same are known, and they are generally obtained from the reaction of aldehydes with primary amines; for more details see R.W. Layer, Chem. Rev. 63, 489-510 (1963).

The reaction of thiols with certain Schiff bases has been reported by G.W. Stacy and R.J. Morath, J. Am. Chem. Soc. 74, 3885-6 (1952), G.W. Stacy et al, ibid. 77, 3869-72 (1955). More recent studies have described the characteristics of this reaction and are found in the following references: T.R. Oakes and G.W. Stacy, J. Am. Chem. Soc. 94, 1594-1700 (1972); T.R. Oakes, Diss. Abs. 25, 1552-3 (1964); also Univ. Microfilms No. 64-5803, 66 pp (1963); Y. Ogata and A. Kawasaki, J. Chem. Soc. Perkin Trans. II, 134-139 (1975).

In general, these studies reveal the following typical characteristics for the addition of a thio to a Schiff base to form the thio adduct:

(a) addition takes place rapidly in solution,

(b) equilibrium is attained rapidly,

(c) the addition reaction is one of mobile equilibrium, and

(d) some thio adducts are stable enough to be isolated in pure form but others are not (cf. also A. Martani C.A. 48, 12737e (1954) wherein a number of such adducts are reported as being isolatable, yet; some decompose even upon crystallization).

The use of the adduct or intermediate for making the compounds of this invention comprises reacting said adduct with an iso(thio)cyanate contemplated herein. Stacy et al, J. Org. Chem. 23, 1760-1764 (1958) teaches that such adducts do not react with iso(thio)cyanates to form ureas. Specifically, Stacy et al discloses the reaction of $R'CH(SR^{2'})NHR^{1'}$ wherein $R'$ and $R^{1'}$ are both phenyl, and $R^{2'}$ is p-tolyl, with phenyl isocyanate, however, the anticipated urea compound was not formed.

It has now been surprisingly and unexpectedly found that the thio adducts of the instant invention readily produce the desired substituted ureas when contacted with the iso(thio)cyanates indicated above. Excellent yields are obtained especially when the iso(thio)cyanate $R^3NCX$ is added to the reaction mixture containing the $RCH(SR^2)NHR^1$ adduct of this invention in equilibrium with the thio $R^2SH$ and Schiff base $RCH=NR^1$, wherein R, $R^1$, $R^2$, $R^3$ and X have the meanings above. Although no real explanation can be provided, it appears that the reaction of the iso(thio)cyanates with the adducts shifts the equilibrium away from the starting thiols and Schiff bases, thus leading to the ureas of this invention.

Such adducts, and for that matter, those equilibrium reaction mixtures, are new, and their usefulness for making the substituted urea chemicals of this invention is unexpected. In addition, they are distinct and different from adducts known, and so are their chamical characteristics.

In general, the process of the present invention is carried out in the following manner:

Having prepared the desired Schiff base $RCH=NR^1$ according to well known procedures such as reacting an aldehyde of the structural formula RCHO with a primary amine $H_2NR^1$, wherein R and $R^1$ have the above meanings, the dry Schiff base is dissolved in a suitable, dry, aprotic solvent such as tetrahydrofuran, benzene, toluene, octane, decane, dodecane, etc., and while stirring, the thiol is added to the Schiff base at approximately stoichiometric amounts or with a slight deficiency or excess of thiol. Conversely, the thiol may be first dissolved in a solvent and the Schiff base added thereto. Preferably, the reaction is conducted under a dry atmosphere such as dry $N_2$. The reaction temperature is not particularly critical, and may vary from about -10°C to 75°C, preferably from 0°C to 30°C, usually ambient (e.g. room temperature) conditions suffice. The thiol or Schiff base may be added to the solution of the other fairly readily over a relatively short period of time as long as the slightly exothermic reaction is kept at temperatures ranging from -10°C to 75°C, usually 0°C to 30°C. Although as mentioned above, the resultant adduct in some instances may be isolated, it is advantageous to use the thiol-Schiff base-adduct reaction mixture for the preparation of the substituted ureas by adding to said reaction mixture the above defined iso(thio)cyanate, usually at a slight molar excess, i.e., at an iso(thio)cyanate/adduct or thiol or Schiff base molar ratio of 1/1-1.2/1, preferably 1/1-1.1/1, usually 1/1-1.05/1 at temperatures ranging from about -10° to 75°C, preferably 0°C to 40°C and usually from 5°C to 30°C for about 1 to 20 hours, preferably 2 to 8 hours, and most preferably for about 2 to 5 hours. After the iso(thio)cyanate addition, stirring may be continued for an additional time length such as overnight (ca. 16 hours) or less and, finally, this reaction mixture may optionally be heated to 40-100°C for 0.5-4 hours, preferably 1-3 hours, usually 2 hours of heating is sufficient to bring the reaction to completion. This last step may be carried out with or without any catalyst known for enhancing amine-isocyanate reactions such as dibutyltin diacetate or triethylamine. Less preferably, the iso-(thio)cyanate and Schiff base may be mixed first followed by the addition of the thiol. Least recommended is a procedure, although possible, wherein thiol and iso(thio)cyanate are mixed and then added to the Schiff base.

As mentioned previously, the adduct may be isolated and then reacted with the isocyanate in a suitable solvent to form the urea.

However, a significant advantage of the process is that it may be carried out in a single vessel without isolation of the adduct. A further advantage is that it is effective for preparing ureas from Schiff base-thiol adducts which are often not stable enough to be isolated in pure form due in part to the equilibrium nature of the addition reaction. The fact that the adduct is formed in a rapidly attained mobile equilibrium reaction allows for efficient conversion of thiol and Schiff base through the adduct intermediate even if said adduct is unstable to form the desired substituted urea. Although not required, in practice the Schiff bases employed are usually stable enough to make it convenient to isolate them prior to further reaction. The thiol is then added to the Schiff base in the presence of a suitable solvent, followed by addition of the iso-(thio)cyanate as described above in general and specifically in the examples below. The entire process may be carried out in one vessel starting with the desired materials.

The resultant substituted urea products may be isolated and purified by any one of several convenient methods. In most cases, addition of a non-solvent, e.g. petroleum ether, often precipitates the urea product which is then filtered, dried, and if necessary, recrystallized from solvents or solvent mixtures, e.g., benzene, cyclohexane, ethanol, methanol, isopropanol, ethanol-water, tetrahydrofuran-benzene, tetrahydrofuran-petroleum ether or benzene-petroleum ether. The products are usually crystalline solids, but in some cases oils are obtained.

The substituted ureas obtained by the above described process are useful as pre- and/or postemergence herbicides.

Compounds within the spirit and contemplation of this invention are disclosed in Table I below. Certain abbreviations are used in said Table for reasons of simplicity, namely, "n" stands for "normal," and "c" means "cyclo."

## Table I

Structural Formula: $R^2SCH(R)N(R^1)C(X)NHR^3$

| R | $R^1$ | $R^2$ | $R^3$ | X |
|---|---|---|---|---|
| | | **Group "A" Compounds** | | |
| furfuryl | $CH_3$ | $CH_3$ | $2\text{-}F\text{-}C_6H_4$ | 0 |
| $CH_2CH=CH_2$ | " | " | " | 0 |
| $CH(CH_3)_2$ | $CH_2CH=CH(n\text{-}C_3H_7)$ | " | " | 0 |
| " | $C(CH_3)_2C=CH$ | " | " | 0 |
| $CH(CH_3)CH(CH_3)C_2H_5$ | $CH_3$ | $n\text{-}C_{18}H_{37}$ | " | 0 |
| $n\text{-}C_8H_{17}$ | " | $c\text{-}C_6H_{11}$ | " | 0 |
| $CH_3$ | " | $CH_2(CH_2)_7O(n\text{-}C_6H_{13})$ | " | 0 |
| $C_2H_5$ | " | $CH_2CH_2COO(n\text{-}C_{18}H_{37})$ | " | 0 |
| $CH_3$ | " | $CH_2(CH_2)_3OOCNH(n\text{-}C_8H_{17})$ | " | 0 |
| " | " | $CH_2CH_2N(n\text{-}C_8H_{17})_2$ | " | 0 |
| $CH(CH_3)_2$ | " | see footnote (1) | " | 0 |
| " | " | $4\text{-}(CH_3)_3C\text{-}C_6H_4$ | " | 0 |
| " | " | $4\text{-}n\text{-}C_4H_9O\text{-}C_6H_4$ | " | 0 |
| | | **Group "B" Compounds** | | |
| $3,4\text{-}(CH_3)_2\text{-}C_6H_3$ | $CH_2CH=CH(n\text{-}C_3H_7)$ | $CH_3$ | " | 0 |
| " | $C(CH_3)_2)C=CH$ | " | " | 0 |
| " | $c\text{-}C_3H_5$ | $n\text{-}C_{12}\text{-}H_{25}$ | " | 0 |
| " | $CH_3$ | $CH_2(CH_2)_7O(n\text{-}C_6H_{13})$ | " | 0 |
| " | " | $CH_2CH_2COO(n\text{-}C_{18}H_{37})$ | " | 0 |
| " | " | $CH_2CH(C_2H_5)N(n\text{-}C_8H_{17})_2$ | " | 0 |
| $4\text{-}CH_3O\text{-}C_6H_4$ | " | $CH_2(CH_2)_3OOCNH(n\text{-}C_8H_{17})$ | " | 0 |
| " | " | $CH_2CH_2OOCNH(c\text{-}C_6H_{11})$ | " | 0 |
| " | " | $CH_2CH(C_2H_5)N(CH_2)_4$ | " | 0 |
| $2\text{-}CH_3O\text{-}3,6\text{-}Cl_2C_6H_2$ | " | $CH_3$ | " | 0 |
| | | **Group "C" Compounds** | | |
| 2-furyl | $CH_2CH=CH(n\text{-}C_3H_7)$ | $4\text{-}CH_3O\text{-}C_6H_4$ | " | 0 |
| " | $C(CH_3)_2C=CH$ | " | " | 0 |

---

(1) $CH(n\text{-}C_6H_{13})CH_2\text{-}(4\text{-morpholinyl})$

The preparation of the substituted ureas of this invention is illustrated by the following examples. It should be noted that the compound numbers refer to chemicals summarized in Tables II (A compounds), III (B compounds) and IV (C compounds), and the subsequent tables V-X demonstrating the herbicidal activities are referring to the compound A-, B- and C- numbers, respectively.

## Example 1

(a)  Preparation of N-(2-methylpropylidene) cyclopentanamine (Schiff base)

To a 48.5 g (0.57 mol) of cyclopentylamine cooled in an ice bath was added with stirring, 44 g (0.60 mol) if isobutyraldehyde. After the initial vigorous exothermic reaction had subsided (15 minutes), approximately 36 g of 3A molecular sieve was added, the reaction mixture allowed to come to room temperature and stirred for 2 hours. The sieve was removed by vacuum filtration to yield 64.5 g (81% of theory) of the title compound as a clear liquid. The infrared spectrum showed an absorption at 1675 $cm^{-1}$ (C=N) and the absence of carbonyl (C=O) absorption at 1720 $cm^{-1}$. The NMR (proton nuclear magnetic resonance) spectrum confirmed the structure assignment.

(b)  Preparation of N-cyclopentyl-N'-(2-fluorophenyl)-N-[(2-methyl-1-methylthio) propyl] urea (compound No. A-31)

To a stirred solution of 4.8 g (0.10 mol) of methanethiol dissolved in 150 ml of dry tetrahydrofuran and cooled in a bath at approximately 10°C was added 14.6 g (0.105 mol) of N-(2-methylpropylidene)cyclopentanamine of Example 1(a) with 20 ml of dry tetrahydrofuran. The mixture was stirred ina stoppered flask at 5° to 10°C for 3 hours followed by dropwise addition of a solution of 14.6 g (0.105 mol) of 2-fluorophenyl isocyanate dissolved in 100 ml of dry tetrahydrofuran over a period of 5 hours. During the addition the reaction mixture was cooled in a bath at approximately 5° to 10°C. After the addition was completed, the reaction mixture was allowed to warm to room temperature and stirred overnight.

The following day, 3 drops of dibutyltin diacetate catalyst was added and the mixture stirred for 2 hours at room temperature followed by heating at approximately 40°C for 2 hours. Next, the solvent was removed at reduced pressure to yield 29.4 g of the title compound as a viscous oil. The infrared spectrum showed absorptions at 3250 cm$^{-1}$ (NH) and 1670 cm$^{-1}$ (urea C=O) consistent with the assigned structure. The NMR spectrum confirmed the structure assignment.

Analysis:      for $C_{17}H_{25}FN_2OS$ (percent):
Calculated:    C: 62.93; H: 7.77; N: 8.64; S: 9.88
Found:         C: 61.41; H: 7.22; N: 8.40; S: 10.00.

Example 2

Preparation of N'-(2-fluorophenyl)-N-methyl-N-[(2-methyl-1-methylthio)propyl] urea (Compound No. A-5)

To a stirred solution of 4.8 g (0.1 mol) of methanethiol dissolved in 100 ml of dry tetrahydrofuran was added 8.5 g (0.1 mol) of N-(2-methylpropylidene) methanamine (cf. Tetrahedron 24, 3907-22 (1968) for the preparative method used and C.A. 42, 8156b for the properties). The mixture was stirred at room temperature in a well stoppered flask for 1 hour, after which 3 drops of dibutyltin diacetate catalyst was added followed by dropwise addition of a solution of 13.7 g (0.1 mol) of 2-fluorophenyl isocyanate in 110 ml of dry tetrahydrofuran over a period of 2.5 hours. The mixture was stirred at room temperature overnight and then heated at about 40°C for 2.5 hours. The volume was reduced to about 50 ml by sweeping N$_2$ over the warmed solution. Addition of petroleum ether and cooling resulted in precipitation of a fine white solid which was collected by vacuum filtration, washed with petroleum ether and dried to yield 10.1 g of white solid. Recrystallization from isopropanol-water mixture yielded 8.9 g of the title compound, m.p. 90-91.5°C. The infrared spectrum showed absorptions at 3250 cm$^{-1}$ (NH) and 1635 cm$^{-1}$ (urea C=O) consistent with the assigned structure. The NMR spectrum confirmed the assigned structure.

Analysis:      for $C_{13}H_{19}FN_2OS$ (percent):
Calculated:    C: 57.75; H: 7.08; N: 10.36; S: 11.86
Found:         C: 57.82; H: 7.07; N: 10.34; S: 11.77.

## Example 3

### Preparation of N-methyl-N-[(1-(cyclohexyl)-1-(methylthio))-methyl]-N'-(2-fluorophenyl) urea (Compound No. A-39)

To a stirred solution of 6.3 g (0.05 mol) of N-(cyclohexylmethylene) methanamine (cf. Tetrahedron 24, 3907-22 (1968) for preparative method) dissolved in 80 ml of dry tetrahydrofuran was added .32 ml of 1.6 M solution of methanethiol in dry tetrahydrofuran. The mixture was stirred at room temperature in a well-stoppered flask for 1 hour and 20 minutes followed by dropwise addition of a solution of 6.9 g (0.05 mol) of 2-fluorophenyl isocyanate in 110 ml of dry tetrahydrofuran over a period of 3 years. The reaction mixture was stirred at room temperature overnight, and then 3 drops of dibutyltin diacetate catalyst was added, and the mixture heated at about 50°C for 1.75 hours with stirring. The mixture was then brought to a boil, and the volume reduced to about 30 ml by sweeping nitrogen over the heated solution. While boiling, about 120 ml of petroleum ether was added, and the resulting clear solution, upon cooling to room temperature, formed a solid precipitate which was collected by suction filtration, washed with petroleum ether and dried to give 10.1 g of the title compound, m.p. 103-108°C. The infrared spectrum showed absorptions at 3220 cm$^{-1}$ (NH) and 1635 cm$^{-1}$ (urea C=O) consistent with the assigned structure. The NMR spectrum confirmed the assigned structure.

| Analysis: | for C$_{16}$H$_{23}$FN$_2$OS (percent): |
| --- | --- |
| Calculated: | C: 61.90; H: 7.47; N: 9.03; S: 10.53 |
| Found: | C: 61.98; H: 7.58; N: 8.48; S: 8.96. |

## Example 4

### Preparation of N'-cyclohexyl-N-methyl-N-[(2-methyl-1-methylthio) propyl] urea (Compound No. A-1)

To a stirred solution of 10.0 g (0.208 mol) of methanethiol dissolved in 200 ml of dry tetrahydrofuran and cooled in a bath at 10° to 15°C was added 19.1 g (0.224 mol) of N-(2-methylpropylidene) methanamine (cf. Example 2). The mixture was stirred in a stoppered flask at 10° to 15°C for 2 hours followed by the dropwise addition of a solution of 27.8 g (0.22 mol) of cyclohexyl isocyanate in 75 ml of dry tetrahydrofuran over a period of 4.5 hours. During the addition the reaction mixture was stirred at 10° to 15°C. After the addition was complete, the reaction mixture was allowed to warm to room temperature and stirred overnight.

The following day, 5 drops of dibutyltin diacetate catalyst was added and the mixture stirred at room temperature for 2 hours followed by heating at approximately 40°C for 2 hours. Next, the volume of the reaction mixture was reduced by approximately 50% by sweeping $N_2$ over the warmed reaction mixture. Addition of petroleum ether and cooling resulted in the preciptation of a white solid which was collected by vacuum filtration and dried to yield 11.2 g of the title compound, m.p. 117°-118°C. The filtrate yielded a second crop of 12.2 g of the title compound, m.p. 105°-108°C. The infrared spectrum showed absorptions at 3300 $cm^{-1}$ (NH) and 1650 $cm^{-1}$ (urea C=O) consistent with the assigned structure. The NMR spectrum confirmed the structure assignment.

Analysis:     for $C_{13}H_{26}FN_2OS$ (percent):
Calculated:   C: 60.42; H: 10.14; N: 10.84; S: 12.41
Found:        C: 61.19; H: 10.39; N: 10.87; S: 12.11.

## Example 5

### Preparation of N'-(2-fluorophenyl)-N-[(2-methyl-1-methylthio) propyl]-N-(phenylmethyl) urea (Compound No. A-3

To a stirred solution of 4.8 g (0.10 mol) of methanemethiol dissolved in 150 ml of dry tetrahydrofuran and cooled to approximately 10°C was added 16.1 g (0.10 mol) of N-(2-methylpropyli-dene) benzenemethanamine (prepared by the method of Example 1(a), and cf. C.A. 42, 8157c for properties and an alternate method of preparation). The mixture was stirred in a stoppered flask at approximately 10°C for 2 hours followed by the dropwise addition of a solution of 14.1 g (0.10 mol) 2-fluorophenyl isocyanate in 100 ml of dry tetrahydrofuran over a period of 4.3 hours. During the addition the reaction mixture was cooled at approximately 6° to 10°C. After the addition was complete, the reaction mixture was allowed to warm to room temperature and stirred overnight.

The following day, 3 drops of dibutyltin diacetate catalyst was added and the mixture was stirred for 2 hours at room temperature and then heated at approximately 40°C for 2 hours. The usual work-up involving removal of the solvent at reduced pressure yielded 33.5 g of the title compound as a viscous oil. The infrared and NMR spectra confirmed the structure assignment.

Analysis:       for $C_{19}H_{23}FN_2OS$ (percent):
Calculated:     C: 65.86; H: 6.69; N: 8.09; S: 9.26
Found:          C: 66.15; H: 6.69; N: 7.66; S: 9.03.

Example 6

Preparation of N'-(2-fluorophenyl)-N-methyl-N-[(2-methyl-1-methylthio)propyl] thiourea (Compound No. A-6)

To a stirred solution of 4.4 g (0.092 mol) of methanethiol dissolved in 100 ml of dry tetrahydrofuran and cooled in a bath at approximately 10°C was added 8 g (0.094 mol) of N-(2-methylpropylidene) methanamine (cf. Example 2). The mixture was stirred in a stoppered flask in a cooling bath at 0° to 10°C for 2.5 hours followed bh the dropwise addition of a solution of 14.4 g (0.094 mol) of 2-fluorophenyl isothiocyanate in 100 ml of dry tetrahydrofuran over a period of 7 hours. During the addition the reaction mixture was cooled in a bath at 10° to 20°C. After the addition was completed, the reaction mixture was allowed to warm to room temperature and stirred overnight.

The following day, 3 drops of dibutyltin diacetate catalyst was added and the mixture stirred at room temperature for 1.5 hours followed by heating at approximately 40°C for 2.5 hours. The mixture was then cooled to room temperature and stirred overnight.

Next, the solvent was removed by sweeping $N_2$ over the heated reaction mixture to yield a viscous oil.

Subsequent treatment of this oil with toluene and hexane produced a crude solid product which upon rectystallization from isopropanol-water followed by a second recrystallization from ethanol-water (cold) yielded 5.9 g of the title compound as a pale yellow solid, m.p. 97°-98.5°C. The infrared and NMR spectra confirmed the assigned structure.

Analysis: for $C_{13}H_{19}FN_2S_2$ (percent):
Calculated: C: 54.51; H: 6.69; N: 9.78; S: 22.39
Found: C: 54.49; H: 6.70; N: 9.84; S: 22.32.

## Example 7

Preparation of N'-(2-fluorophenyl)-N-[(1-(2-hydroxyethylthio)-2-methyl)propyl]-N-methyl urea (Compound No. A-17)

To a stirred solution of 15.6 g (0.20 mol) of 2-mercaptoethanol dissolved in 150 ml of dry tetrahydrofuran at room temperature was added 17.9 g (0.21 mol) of N-(2-methylpropylidene) methanamine (cf. Example 2). The mixture was stirred at room temperature for 3 hours followed by the dropwise addition at room temperature of a solution of 28.3 g (0.206 mol) of 2-fluorophenyl isocyanate in 90 ml of dry tetrahydrofuran over a period of 8 hours. After the addition was complete, the reaction mixture was stirred at room temperature for 12 hours. Next, 3 drops of dibutyltin diacetate catalyst was added and the mixture stirred for 2 hour at room temperature and then for 2 hours at approximately 40°C. Removal of the solvent in vacuo yielded 57.7 g of the title compound as a viscous oil. The infrared and NMR spectra confirmed the assigned structure.

Analysis: for $C_{14}H_{21}FN_2O_2S$ (percent):
Calculated: C: 55.97; H: 7.05; N: 9.33; S: 10.67
Found: C: 55.95; H: 6.96; N: 9.70; S: 10.15.

## Example 8

Preparation of N-[(1-(2-(cyclohexylaminocarbonyloxy)ethylthio)-2-methyl)propyl]-N'-(2-fluorophenyl)-N-methyl urea (Compound No. A-23)

To a stirred solution of 15.0 g (0.05 mol) of N'-(2-fluorophenyl)-N-[(1-(2-hydroxyethylthio)-2-methyl)propyl]-N-methyl urea of Example 7 dissolved in 150 ml of dry tetrahydrofuran at room temperature was added 6.7 g (0.053 mol) of cyclohexyl isocyanate and 3 drops of dibutyltin diacetate catalyst. The reaction mixture was stirred overnight at room temperature under anhydrous conditions. Next, the reaction mixture was heated at 40°C for 3 hours and then refluxed for 5 hours. The mixture was then allowed to cool to room temperature and stirred overnight.

The following day, the mixture was concentrated to approximately 100 ml by sweeping $N_2$ over the warmed reaction mixture. Addition of 350 ml of petroleum ether and cooling in an ice bath precipitated a small amount (1.3 g) of a solid impurity which was removed by filtration. The filtrate was evaporated under vacuum to yield 21.6 g of the title compound as a viscous oil. The infrared spectrum showed absorptions at 3375 cm$^{-1}$ (NH), 1730 cm$^{-1}$ (carbamate C=O) and at 1675 cm$^{-1}$ (urea C=O). The NMR spectrum confirmed the assigned structure.

| Analysis: | for $C_{21}H_{32}FN_3O_3S$ (percent): |
|---|---|
| Calculated: | C: 59.26; H: 7.58; N: 9.88; S: 7.53 |
| Found: | C: 60.01; H: 7.99; N: 9.69; S: 6.57. |

## Example 9

### Preparation of N'-(2-fluorophenyl)-N-[(2-methyl-1-methylthio)-propyl]-N-2-propenyl urea (Compound No. A-28)

To a stirred solution of 4.8 g (0.10 mol) of methanethiol dissolved in 150 ml of dry tetrahydrofuran and cooled to approximately 10°C was added dropwise over a 10 minute period 11.1 g (0.10 mol) of N-(2-methylpropylidene)-2-propen-1-amine (prepared by the method of Example 1(a) and cf. C.A. 42, 8156i for properties and an alternate preparative procedure) follwed by the addition of 20 ml of dry tetrahydrofuran. The mixture was stirred in a stoppered flask at 5° to 10°C for 2 hours followed by the dropwise addition of a solution of 14.4 g (0.105 mol) of 2-fluorophenyl isocyanate in 100 ml of dry tetrahydrofuran over a period of 3.75 hours. During the addition, the reaction mixture was stirred at approximately 10°C. After the addition was complete, the reaction mixture was allowed to warm (gradually) to room temperature and stirred overnight.

The following day, 3 drops of dibutyltin diacetate catalyst was added and the mixture was stirred for 2 hours at room temperature and then heated at approximately 40°C and for 2 hours. Next the solvent was removed at reduced pressure on a rotary evaporator and finally at a pressure of 0.3 mm of Hg at 65°C to yield 29.3 g of the title compound as a light yellow oil which on standing at room temperature partially solidified. The infrared and NMR spectra confirmed the structure assignment.

Analysis:    for $C_{15}H_{21}FN_2OS$ (percent):
Calculated:    C: 60.78; H: 7.14; N: 9.45; S: 10.82
Found:    C: 60.59; H: 6.91; N: 9.44; S: 10.74.

## Example 10

### Preparation of N-methyl-N'-[4-(1-methylethyl)phenyl]-N-[(2-methyl-1-methylthio)propyl] urea (Compound No. A-61)

To a stirred solution of 3.4 g (0.071 mol) of methanethiol dissolved in 150 ml of dry tetrahydrofuran and cooled at approximately 10°C was added 6.1 g (0.072 mol) of N-(2-methylpropylidene) methanamine (cf. Example 2). The mixture was stirred in a stoppered flask at approximately 10°C for 2.5 hours followed by the dropwise addition of a solution of 11.6 g (0.072 mol) of 4-(1-methylethyl) phenyl isocyanate in 100 ml of dry tetrahydrofuran over a period of 4 hours. During the addition, the reaction mixture was cooled at approximately 10°C. After the addition was complete, the reaction mixture was allowed to warm to room temperature and stirred overnight.

The following day, 3 drops of dibutyltin diacetate catalyst was added and the mixture stirred at room temperature for 2 hours followed by heating at approximately 40°C for 2 hours. Next, the volume of the mixture was reduced to approximately 50 ml by sweeping $N_2$ over the warmed reaction mixture. Addition of petroleum ether and cooling resulted in precipitation of a white solid which was collected by vacuum filtration, washed with petroleum ether and dried to yield 13.9 g of the title compound, m.p. 134.5°-137°C. The infrared spectrum showed absorptions at 3250 cm$^{-1}$ (NH) and 1630 cm$^{-1}$ (urea C=O) consistent with the assigned structure. The NMR spectrum confirmed the structure assignment.

Analysis:        for $C_{16}H_{26}N_2OS$ (percent):
Calculated:      C: 65.26; H: 8.90; N: 9.52; S: 10.89
Found:           C: 65.37; H: 8.55; N: 9.46; S: 10.91.

## Example 11

Preparation of N-methyl-N-[(1-methylthio-1-(3,4-methylenedioxy) phenyl))methyl]-N'-(2-fluorophenyl) urea (Compound No. B-46)

To a stirred solution of 9.7 g (0.06 mol) of N-[(3,4-(methylene dioxy)phenyl)methylene]methanamine (cf. J.Org. Chem. 43, 2854 (1978)) in 120 ml of dry tetrahydrofuran was added 40 ml of a 1.5M solution of methanethiol in dry tetrahydrofuran. The reaction mixture was stirred at room temperature for 2 hours with continued stirring followed by dropwise addition of a solution of 8.3 g of 2-fluorophenyl isocyanate in 125 ml of dry tetrahydrofuran over a period of about 5 hours. The mixture was stirred at room temperature overnight and then 3 drops of dibutyltin diacetate catalyst were added followed by continued stirring and heating at 50°C for about 2.5 hours. At this point, addition of petroleum ether precipitated a solid which was collected by vacuum filtration and dried. Recrystallization from an ethanol-water mixture gave 12.5 g of the title compound, m.p. 88-92°C. The infrared spectrum showed absorption at 3260 cm$^{-1}$ (NH) and 1640 cm$^{-1}$ (urea C=O) consistent with the assigned structure. The NMR spectrum confirmed the assigned structure.

Analysis:        for $C_{16}H_{17}FN_2O_3S$ (percent):
Calculated:      C: 58.60; H: 4.92; N: 8.04; S: 9.20
Found:           C: 58.13; H: 4.84; N: 8.68; S: 9.69.

## Example 12

Preparation of N-methyl-N-[1-(cyclopentylthio)phenylmethyl]-N'-(2-fluorophenyl) urea (Compound No. B-13)

To a solution of 7.1 g (0.06 mol) of N-benzylidenemethylamine dissolved in 100 ml of dry toluene was added 6.2 g (0.06 mol) of cyclopentanethiol and the mixture stirred for 1.25 hours at room temperature. At the end of this time, a solution of 8.2 g (.06 mol) of 2-fluorophenyl isocyanate in 100 ml of dry toluene was added dropwise over a period of 3 hours. The reaction mixture was then stirred at room temperature overnight. The mixture was then heated at about 50°C for 2 hours. Upon cooling to room temperature, a small quantity of white precipitate formed which was removed by filtration and discarded. Upon evaporation of residual solvent the filtrate formed a waxy solid which was recrystallized from a small volume of a mixture of cyclohexane-petroleum ether resulting in 13 g of the title compound, m.p. 87.5-89.5°C. The infrared spectrum showed absorption at 3230 cm$^{-1}$ (NH) and 1640 cm$^{-1}$ (urea C=O) consistent with the assigned structure. The NMR spectrum confirmed the assigned structure.

| Analysis: | for $C_{20}H_{23}FN_2OS$ (percent): |
|---|---|
| Calculated: | C: 67.01; H: 6.47; N: 7.82; S: 8.95 |
| Found: | C: 66.77; H: 6.54; N: 8.22; S: 8.21. |

## Example 13

### Preparation of N-methyl-N-[(1-(4-methoxyphenyl)-1-methylthio)methyl]-N'-(2-fluorophenyl) urea (Compound No. B-31)

To a stirred solution of 2.4 g (0.05 mol) of methanethiol dissolved in 100 ml of dry tetrahydrofuran was added a solution of 7.5 g (0.05 mol) of N-[(4-methoxyphenyl)methylene]methanamine (see Org. Syn. Coll. IV p. 605 (1963)), in 25 ml of dry tetrahydrofuran. The resulting mixture was stirred for 2 hours at room temperature followed by dropwise addition of a solution of 6.9 g (0.05 mol) of 2-fluorophenyl isocyanate in 110 ml of dry tetrahydrofuran over a period of 2.5 hours. The reaction mixture was stirred at room temperature overnight. At this time the mixture was heated at 50°C for 1 hour. The mixture was then heated to a boil and while boiling about 300 ml of petroleum ether was added. The resultant solution was cooled at about -10°C overnight yielding a crystalline solid, which, after collection by vacuum filtration and drying, gave 10 g of the title compound, m.p. 79.5-82°C. The infrared spectrum showed absorptions at 3280 cm$^{-1}$ (NH) and 1640 cm$^{-1}$ (urea C=O) consistent with the assigned structure. The NMR spectrum confirmed the structure assignment.

Analysis:      for $C_{17}H_{19}FN_2O_2S$ (percent):
Calculated:    C: 61.05; H: 5.73; N: 8.38; S: 9.59
Found:         C: 60.94; H: 5.66; N: 8.55; S: 8.83.

Example 14

Preparation of N-methyl-N-[1-(2-diethylaminoethylthio)
phenylmethyl]-N'-(2-fluorophenyl) urea (Compound No. B-21)

To a mixture of 17 g (0.1 mol) of 2-diethylaminoethanethiol hydrochloride in 150 ml of dry tetrahydrofuran was added to 10.5 g of triethylamine, and the mixture stirred for 10 minutes. At the end of this time, 11.9 g (0.1 mol) of N-benzylidenemethylamine was added, and the resulting mixture was stirred for 3 hours at room temperature. Then, a solution of 14 g of 2-fluorophenyl isocyanate dissolved in 100 ml of dry tetrahydrofuran was added dropwise with continuous stirring over a period of 4 hours. The reaction mixture was stirred at room temperature overnight. At this point, 2 drops of dibytyltin diacetate catalyst was added and the reaction mixture was heated at 50°C for 1.5 hours while stirring. The mixture was cooled to room temperature, and the solid triethylammonium hydrochloride was removed by filtration. Solvent was removed from the filtrate by evaporation to yield an oily residue. The residue was dissolved in a mixture of benzene-petroleum ether and washed in a separatory funnel first with an aqueous 1% sodium hydroxide solution and then two times with distilled water to remove residual triethylammonium chloride. The organic benzene-petroleum ether mixture was dried over anhydrous sodium sulfate, and the solvent was removed by evaporation yielding an oily residue which solidified on treatment with petroleum ether. Attempted recrystallization from a mixture of benzene-petroleum ether yielded only 1 g of crystalline solid identified as N,N'-di-(2-fluorophenyl) urea, a side product formed possibly due to the presence of spurious moisture. The benzene-petroleum ether mother liquor was evaporated to give 16 g of a waxy solid identified as the title compound. Thus, the infrared spectrum showed absorptions at 3190 $cm^{-1}$ (NH) and 1640 $cm^{-1}$ (urea C=O) consistent with the assigned structure. The NMR spectrum also confirmed the structure assignment.

Analysis:      for $C_{21}H_{28}FN_3OS$ (percent):
Calculated:    C: 64.75; H: 7.24; N: 10.79; S: 8.23
Found:         C: 62.98; H: 7.37; N: 10.81; S: 8.42.

## Example 15

(a) Preparation of N-[(1-(4-hydroxyphenylthio)phenylmethyl] methanamine

To a stirred solution of 126 g (1.0 mol) of 4-mercaptophenol dissolved in 800 ml of dry benzene was added 119 g (1.0 mol) of N-benzylidenemetylamine. After a very mild exotherm, the mixture was stirred at room temperature overnight. The reaction mixture was warmed slightly (to about 40°C) for 2 hours and then cooled to 5°C overnight. The while crystalline precipitate which formed was collected by vacuum filtration and dried yielding 166 g of the title compound, m.p.: 81-82.5°C. The volume of the filtrate was reduced to 150 ml by evaporation to yield an additional 35 g of the title compound after collection by filtration and drying. The NMR spectrum confirmed the assigned structure.

Analysis:      $C_{14}H_{15}NOS$ (percent):
Calculated:    C: 68.45; H: 6.16; N: 5.72; S: 13.02
Found:         C: 68.44; H: 6.17; N: 5.59; S: 12.67.

(b) Preparation of N-methyl-N-[1-(4-hydroxyphenylthio)phenyl methyl]-N'-(2-fluorophenyl) urea (Compound No. C-1)

To a solution of 165 g of N-[1-(4-hydroxyphenylthio)phenyl-methyl] methanamine of Example 15(a) dissolved in 500 ml of dry tetrahydrofuran was added a solution of 95.1 g of 2-fluorophenyl isocyanate in 150 ml of dry tetrahydrofuran. Upon mixing, an immediate exothermic reaction occurred, and the temperature rose to 45°C. After stirring for 1.5 hours, the reaction mixture was gently refluxed for 5 hours. The volume of the mixture was then reduced slightly by evaporation until a solid precipitate started to form. The mixture was cooled at 5°C overnight, and the white solid precipitate was collected by vacuum filtration. The solid was washed first with a mixture of 300 ml petroleum ether - 50 ml tetrahydrofuran then with 500 ml petroleum ether. The solid was dried yielding 216 g of the title compound m.p. 153-154.5°C. Evaporation of the filtrate gave additional solid material which after recrystallization from ethanol-water and drying provided an additional 16 g of the title compound. The infrared spectrum showed absorptions at 3310 cm$^{-1}$ (NH), 3210 cm$^{-1}$ (OH) and 1640 cm$^{-1}$ (urea C=O) consistent with the assigned structure. NMR and mass spectra confirmed the assigned structure.

Analysis: for $C_{21}H_{19}FN_2O_2S$ (percent):
Calculated: C: 65.95; H: 5.01; N: 7.33; S: 8.38
Found: C: 65.95; H: 5.13; N: 7.31; S: 8.26.

## Example 16

### Preparation of N-methyl-N-[1-(4-methoxyphenylthio)phenylmethyl]-N'-(2-fluorophenyl) urea (Compound No. C-5)

To a solution of 4.4 g (0.031 mol) of 4-methoxybenzenethiol dissolved in 50 ml of dry benzene was added 3.8 g (0.031 mol) of N-benzylidenemethylamine and 3 drops of dibutyltin diacetate catalyst. The reaction mixture was stirred at room temperature for 1.75 hours followed by dropwise addition of a solution of 4.3 g of 2-fluorophenyl isocyanate dissolved in 75 ml of dry benzene over a period of 2 hours. The reaction mixture was then warmed with stirring at 40°C for 1.5 hours and then was stirred at room temperature overnight. The volume was reduced to about 50 ml by sweeping nitrogen over the warmed reaction mixture. Addition of 150 ml of petroleum ether caused a white solid to precipitate which was collected by vacuum filtration, washed with petroleum ether and dried, yielding 11 g of a solid product. Recrystallization from cyclohexane gave 9.4 g of the title compound, m.p. 124-127.5°C. The infrared spectrum showed absorptions at 3230 cm$^{-1}$ (NH) and 1640 cm$^{-1}$ (urea C=O) consistent with the assigned structure. The NMR spectrum confirmed the structure assignment.

Analysis: for $C_{22}H_{21}FN_2O_2S$ (percent):
Calculated: C: 66.65; H: 5.35; N: 7.07; S: 8.09
Found: C: 66.59; H: 5.48; N: 7.55; S: 7.51.

## Example 17

### Preparation of N-methyl-N-[(1-(4-hydroxyphenylthio)-1-(3,4-dimethoxyphenyl)methyl]-N'-(2-fluorophenyl) urea (Compound No. C-10)

To a solution of 9 g (0.05 mol) of N-[(3,4-dimethoxyphenyl) methylene]methanamine (prepared according to the method disclosed in J. Am. Chem. Soc. $\underline{69}$, 1792 (1947)) dissolved in 150 ml of dry tetrahydrofuran was added 6.3 g (.05 mol) of 4-mercaptophenol. The mixture was stirred at room temperature for 1.5 hours followed by dropwise addition of a solution of 6.9 g (0.05 mol) of 2-fluorophenyl isocyanate in 110 ml of dry tetrahydrofuran over a period of 4.5 hours. The mixture was then stirred at room temperature overnight, 2 drops of dibutyltin diacetate catalyst was added, and the mixture heated at about 40°C for 1 hour. The mixture was then heated to the boiling point, and about 350 ml of petroleum ether added producing a solid precipitate which was collected by vacuum filtration, washed with petroleum ether and dried, yielding 15 g of the title compound, m.p. 159.5-162°C. The infrared spectrum showed absorptions at 3375 cm$^{-1}$ (NH), 3230 cm$^{-1}$ (OH) and 1640 cm$^{-1}$ (urea C=O) consistent with the assigned structure. The NMR spectrum confirmed the structure assignment.

| Analysis: | for $C_{23}H_{23}FN_2O_4S$ (percent): |
|---|---|
| Calculated: | C: 62.43; H: 5.24; N: 6.33; S: 7.25 |
| Found: | C: 62.11; H: 5.27; N: 6.25; S: 7.21. |

## Example 18

### Preparation of N-methyl-N-[(1-(4-hydroxyphenylthio)-1-(2-thienyl)methyl]-N'-(2-fluorophenyl) urea (Compound No. C-17)

To a stirred solution of 6.3 g (0.05 mol) of N-(2-thienylmethylene) methanamine (prepared according to Synthetic Comm. $\underline{4}$ (6), 367-372 (1974)) in 100 ml of dry tetrahydrofuran was added 6.3 g (0.05 mol) of 4-mercaptophenol. The resulting mixture was stirred for 2 hours at room temperature followed by dropwise addition of a solution of 6.9 g (0.05 mol) of 2-fluorophenyl isocyanate in 100 ml of dry tetrahydrofuran over a period of 3.5 hours. The reaction mixture was stirred at room temperature overnight followed by the addition of three drops of dibutyltin diacetate catalyst. The mixture was heated at 40°C with stirring for 1 hour. The volume of the mixture was then reduced to about 50 ml by sweeping nitrogen over the heated solution. While boiling, petroleum ether was added to precipitate a light yellow solid which was collected by suction filtration, washed with petroleum ether and dried to give 19 grams of the title compound, m.p. 109.5-111.5°C. The infrared spectrum showed absorptions at 3200 $cm^{-1}$ (NH), 3100 $cm^{-1}$ (OH) and 1640 $cm^{-1}$ (urea C=O) consistent with the assigned structure. The NMR spectrum confirmed the assigned structure.

| <u>Analysis:</u> | for $C_{19}H_{17}FN_2O_2S_2$ (percent): |
|---|---|
| <u>Calculated:</u> | C: 58.74; H: 4.41; N: 7.21; S: 16.51 |
| <u>Found:</u> | C: 59.54; H: 4.95; N: 6.68; S: 15.17. |

Following essentially the process generally outlined above and specifically in Examples 1-18, additional chemicals were synthesized. These compounds as well as the compounds of Examples 1-18 are summarized in Tables II-IV below.

Each compound summarized in Tables II-IV was characterized by its proton nuclear magnetic resonance spectrum (NMR). These tables display a chemical shift value ($\delta$) for the specified proton or protons of each compound. In Table II, the $\delta$ value shown for each compound is for its characteristic methine proton (C<u>H</u>) represented in the structural formula $R^2SC\underline{H}(R)N(R^1)C(X)NHR^3$. In Tables III and IV the $\delta$ value shown for each compound is either the value for this methine proton (C <u>H</u>) or (where indicated) for the specific proton(s) underlined in the appropriate structural moiety shown for that compound in the Table.

The chemical shifts ($\delta$) were measured either at 60 MHz or at 100 MHz (indicated by an asterisk) in the solvent mentioned and versus tetrametylsilane (TMS) as the internal standard ($\delta$= 0 ppm). The solvents used were deuterochloroform ($CDCl_3$), hexadeutero-acetone ($CD_3COCD_3$) and hexadeuterodimethylsulfoxide ($CD_3SOCD_3$). Each $\delta$ value is followed by a single letter abbreviation indicating the character or apparent splitting pattern of the resonance at that chemical shift. The meaning of these letters is as follows: s (singlet), d (doublet), t (triplet), q (quartet), m (multiplet), b (broad).

Furthermore, it should be noted that certain abbreviations are used in the Tables, namely, m.p. (melting point; single m.p.'s are based on differential thermal analysis (DTA), v (viscous), c (cyclo) and dec. (decomposed upon heating).

## Table II

### Group "A" Compounds

Structural Formula: $R^2SCH(R)N(R^1)C(X)NHR^3$

| Compound A- No. | R | $R^1$ | $R^2$ | $R^3$ | X | m.p. $^0$C | δ (Solvent) |
|---|---|---|---|---|---|---|---|
| 1 | $CH(CH_3)_2$ | $CH_3$ | $CH_3$ | $c-C_6H_{11}$ | O | 117-118 | 5.22d ($CDCl_3$) |
| 2 | " | " | " | $C_6H_5$ | O | 93-98 | 5.12d ($CDCl_3$) |
| 3 | " | $CH_2C_6H_5$ | " | $2-F-C_6H_4$ | O | oil | 5.05d ($CDCl_3$) |
| 4 | $CH_3$ | $CH_3$ | " | " | O | 87-89 | 5.64q ($CDCl_3$) |
| 5 | $CH(CH_3)_2$ | " | " | " | O | 90-91.5 | 5.18d ($CDCl_3$) |
| 6 | " | " | " | " | S | 97-98.5 | 6.3b ($CDCl_3$) |
| 7 | " | " | $C_2H_5$ | " | O | 84-85 | 5.33d ($CD_3COCD_3$) |
| 8 | $CH_3$ | " | $n-C_4H_9$ | " | O | 47 | 5.80q* ($CDCl_3$) |
| 9 | $CH(CH_3)_2$ | " | $n-C_{12}H_{25}$ | " | O | v. oil | 5.17d ($CDCl_3$) |
| 10 | " | " | $CH_2C(CH_3)=CH_2$ | " | O | 94.5-97.5 | 5.10d ($CDCl_3$) |
| 11 | " | " | $c-C_6H_{11}$ | " | O | 70-82 | 5.24d ($CDCl_3$) |
| 12 | " | " | $CH(CH_3)C_6H_5$ | " | O | 110-112 | 5.26d ($CDCl_3$) |
| 13 | " | " | $C_6H_5$ | " | O | 94-104 | 5.63d ($CDCl_3$) |
| 14 | " | " | $4-CH_3-C_6H_4$ | " | O | 102-112 | 5.40d ($CDCl_3$) |
| 15 | " | " | $4-Cl-C_6H_4$ | " | O | 132-135 | 5.57d ($CDCl_3$) |

0093610

Table II - continued

| Compound A- No. | R | R$^1$ | R$^2$ | R$^3$ | X | m.p. $^{o}$C | $\delta$ (Solvent) |
|---|---|---|---|---|---|---|---|
| 16 | CH(CH$_3$)$_2$ | CH$_3$ | 4-CH$_3$O-C$_6$H$_4$ | 2-F-C$_6$H$_4$ | O | 112.5-113.5 | 5.39d* (CDCl$_3$) |
| 17 | " | " | CH$_2$CH$_2$OH | " | O | oil | 5.28d (CDCl$_3$) |
| 18 | " | " | CH$_2$CH$_2$OC$_2$H$_5$ | " | O | 67-71 | 5.25d (CDCl$_3$) |
| 19 | " | " | CH$_2$COOCH$_3$ | " | O | oil | 4.6m (CDCl$_3$) |
| 20 | " | " | CH$_2$CH$_2$COOCH$_3$ | " | O | 80-84 | 5.31d (CDCl$_3$) |
| 21 | " | " | CH$_2$CH$_2$COO(1-C$_8$H$_{17}$) | " | O | oil | 5.39d (CDCl$_3$) |
| 22 | " | " | CH$_2$CH$_2$OOCNHCH$_3$ | " | O | 88 | 5.31d (CDCl$_3$) |
| 23 | " | " | CH$_2$CH$_2$OOCNH(c-C$_6$H$_{11}$) | " | O | oil | 5.28 (CDCl$_3$) |
| 24 | " | " | furfuryl | " | O | 90-92 | 5.37d (CDCl$_3$) |
| 25 | " | " | CH$_2$CH$_2$N(CH$_3$)$_2$ | " | O | v. oil | 5.23d (CDCl$_3$) |
| 26 | " | " | CH(C$_2$H$_5$)CH$_2$N(CH$_2$)$_4$ | " | O | wax | 5.25d (CDCl$_3$) |
| 27 | " | n-C$_4$H$_9$ | CH$_3$ | " | O | v. oil | 5.03d (CDCl$_3$) |
| 28 | " | CH$_2$CH=CH$_2$ | " | " | O | oil | ca. 5.3 (CDCl$_3$) |
| 29 | " | CH$_2$C≡CH | " | " | O | oil | 5.27d (CDCl$_3$) |
| 30 | " | c-C$_3$H$_5$ | " | " | O | oil | 5.05d (CDCl$_3$) |
| 31 | " | c-C$_5$H$_9$ | " | " | O | oil | 5.00d (CDCl$_3$) |

Table II - continued

0093610

-36-

| Compound A- No. | R | R¹ | R² | R³ | X | m.p. °C | δ (Solvent) |
|---|---|---|---|---|---|---|---|
| 32 | $C(CH_3)_3$ | $CH_3$ | $CH_3$ | $2\text{-}F\text{-}C_6H_4$ | 0 | 126 | 5.58s* (CDCl₃) |
| 33 | " | " | $4\text{-}OH\text{-}C_6H_4$ | " | 0 | 160 | 5.69s* (CDCl₃) |
| 34 | $n\text{-}C_6H_{13}$ | " | $CH_3$ | " | 0 | 72.5-75.5 | 5.61t* (CDCl₃) |
| 35 | " | " | $4\text{-}OH\text{-}C_6H_4$ | " | 0 | 128 | 5.74t (CD₃SOCD₃) |
| 36 | $(CH_2)_8CH=CH_2$ | " | $CH_3$ | " | 0 | oil | ca. 5.5 (CDCl₃) |
| 38 | $c\text{-}C_3H_5$ | " | " | " | 0 | 97-99 | 4.84d (CDCl₃) |
| 39 | $c\text{-}C_6H_{11}$ | " | " | " | 0 | 103-108 | 5.36d* (CDCl₃) |
| 40 | " | " | $4\text{-}OH\text{-}C_6H_4$ | " | 0 | 147-148 | 5.32b* (CDCl₃) |
| 41 | 3-cyclohexenyl | " | $CH_3$ | " | 0 | 102-103 | 5.35d (CDCl₃) |
| 42 | $bc\text{-}C_7H_9$ (1) | " | " | " | 0 | oil | 4.95 (CDCl₃) |
| 43 | $CH_2CH(OCH_3)CH_3$ | " | " | " | 0 | oil | 5.6m (CDCl₃) |
| 44 | $CH_2C(CH_3)H(CH_2)_3C(CH_3)_2OCH_3$ | " | " | " | 0 | oil | 5.7m (CDCl₃) |
| 45 | $CH_2CH_2SCH_3$ | " | " | " | 0 | 83-85 | 5.58t (CDCl₃) |
| 47 | $CH_2C_6H_5$ | $CH_3$ | $n\text{-}C_4H_9$ | " | 0 | 68-72.5 | 5.91m* (CDCl₃) |
| 48 | $CH(CH_3)_2$ | " | $CH_3$ | $3\text{-}F\text{-}C_6H_4$ | 0 | 87-90 | 5.14d (CDCl₃) |

Table II - continued

| Compound A- No. | R | R$^1$ | R$^2$ | R$^3$ | X | m.p.$^oC$ | $\delta$ (Solvent) |
|---|---|---|---|---|---|---|---|
| 49 | $CH(CH_3)_2$ | $CH_3$ | $CH_3$ | $4-F-C_6H_4$ | 0 | 110.5-113 | 5.14d $(CDCl_3)$ |
| 50 | " | " | " | $2,4-F_2-C_6H_3$ | 0 | 97-100 | 5.20d $(CDCl_3)$ |
| 51 | " | " | " | $2,5-F_2-C_6H_3$ | 0 | oil | 5.17d $(CDCl_3)$ |
| 52 | " | " | " | $2,6-F_2-C_6H_3$ | 0 | 141.5-143 | 5.18d $(CD_3COCD_3)$ |
| 53 | " | " | " | $2-Cl-C_6H_4$ | 0 | 107.5-109 | 5.25d $(CDCl_3)$ |
| 54 | " | " | " | $.3-Cl-C_6H_4$ | 0 | 101.5-103.5 | 5.16d $(CDCl_3)$ |
| 55 | $CH_3$ | " | " | $4-Cl-C_6H_4$ | 0 | 120-122 | 5.59q $(CDCl_3)$ |
| 56 | $CH(CH_3)_2$ | " | " | $4-Cl-C_6H_4$ | 0 | 127-129 | 5.27d $(CD_3COCD_3)$ |
| 57 | $c-C_3H_5$ | " | " | " | 0 | 125-126 | 4.89d $(CD_3SOCD_3)$ |
| 58 | $CH_2CH_2SCH_3$ | " | " | " | 0 | 93-95 | 5.55t $(CDCl_3)$ |
| 59 | $CH(CH_3)_2$ | " | " | $3,4-Cl_2-C_6H_3$ | 0 | 121-123 | 5.35d $(CD_3COCD_3)$ |
| 60 | " | " | " | $4-CH_3-C_6H_4$ | 0 | 128-130 | 5.17d $(CDCl_3)$ |
| 61 | " | " | " | $4-i-C_3H_7-C_6H_4$ | 0 | 134.5-137 | 5.24d $(CDCl_3)$ |
| 62 | " | " | " | $3-CF_3-C_6H_4$ | 0 | 127-128 | 5.21d $(CD_3COCD_3)$ |
| 63 | " | " | " | $4-CH_3O-C_6H_4$ | 0 | 105-108 | 5.25d $(CD_3 COCD_3)$ |

Table II - continued

| Compound A- No. | R | R¹ | R² | R³ | X | m.p. °C | δ (Solvent) |
|---|---|---|---|---|---|---|---|
| 64 | CH(CH₃)₂ | CH₃ | CH₃ | 4-CH₃S-C₆H₄ | 0 | 110-119 | 5.19d (CDCl₃) |
| 65 | " | " | " | 4-C₆H₅O-C₆H₄ | 0 | 136-145 | 5.17d (CDCl₃) |
| 66 | " | " | " | 4-NO₂-C₆H₄ | 0 | 173-174 | 5.32d (CD₃COCD₃) |
| 67 | " | " | " | 3-CN-C₆H₄ | 0 | 121-123 | 5.15d (CDCl₃) |
| 68 | n-C₁₁H₂₃ | " | " | 2-F-C₆H₄ | 0 | 81-83 | 5.52d (CDCl₃) |
| 69 | bc-C₇H₉ (1) | " | " | 4-i-C₃H₇-C₆H₄ | 0 | 135-137 | 4.93d (CDCl₃) |
| 70 | CH₃ | " | " | " | 0 | 124-125 | 5.64q (CDCl₃) |

Remarks:

(1) bicyclo[2.2.1]hept-5-en-2-yl

## Table III

### Group "B" Compounds

Structural Formula: $R^2SCH(R)N(R^1)C(X)NHR^3$

| Compound B-No. | R | $R^1$ | $R^2$ | $R^3$ | X | m.p. °C | δ (Solvent) |
|---|---|---|---|---|---|---|---|
| 1 | $C_6H_5$ | $CH_3$ | $CH_3$ | $C_6H_5$ | O | 131-133.5 | 2.18s* ($CDCl_3$) |
| 2 | " | " | $CH_3$ | " | S | 116-117.5 | 2.26s* ($CDCl_3$) |
| 3 | " | " | $CH_2CH_2COOCH_3$ | " | O | 111.5-112.5 | 3.65s* ($CDCl_3$) |
| 4 | " | " | $CH_2CH_2N(CH_2CH_3)_2$ | " | O | wax | 1.0m* ($CD_3COCD_3$) |
| 5 | 2-furyl | " | $CH_3$ | " | O | 124-125 | 6.83s* ($CDCl_3$) |
| 6 | " | " | $CH_3$ | " | S | 58-61.5 | 2.26s* ($CDCl_3$) |
| 7 | $C_6H_5$ | " | $CH_2CH_2COOCH_3$ | $4-CH_3-C_6H_4$ | O | 97.5-100.5 | 3.69s* ($CDCl_3$) |
| 8 | $3,4-(CH_3O)_2-C_6H_3$ | " | $CH_3$ | $4-1-C_3H_7-C_6H_4$ | O | 126-130 | 3.80s ($CDCl_3$) |
| 9 | $C_6H_5$ | " | " | $2-F-C_6H_4$ | O | 121.5-122.5 | 6.91s* ($CDCl_3$) |
| 10 | " | $CH_3$ | $CH(CH_3)_2$ | " | O | 80.5-81.5 | 2.98s* ($CDCl_3$) |
| 11 | " | $CH_3$ | $n-C_4H_9$ | " | O | 73.5-75.5 | 6.96s* ($CDCl_3$) |
| 12 | " | " | $C(CH_3)_3$ | " | O | 84-92 | 1.48s* ($CDCl_3$) |
| 13 | " | " | $c-C_5H_9$ | " | O | 87.5-89.5 | 6.98s* ($CDCl_3$) |
| 14 | " | " | $c-C_6H_{11}$ | " | O | 91-96.5 | 6.98s* ($CDCl_3$) |
| 15 | " | " | $CH_2CH_2OH$ | " | O | v.oil | 3.84t* ($CDCl_3$) |
| 16 | " | " | $CH_2CH_2CH_2CH_2OH$ | " | O | v.oil | 3.68m* ($CDCl_3$) |

-39-

0093610

Table III - continued

| Compound B- No. | R | $R^1$ | $R^2$ | $R^3$ | X | m.p. °C | δ (Solvent) |
|---|---|---|---|---|---|---|---|
| 17 | $C_6H_5$ | $CH_3$ | $CH_2COOCH_3$ | $2\text{-}F\text{-}C_6H_4$ | 0 | v.oil | 2.87s* ($CDCl_3$) |
| 18 | " | $CH_3$ | $CH_2CH_2COOCH_3$ | " | 0 | 70-71.5 | 3.68s* ($CDCl_3$) |
| 19 | " | " | $CH_2CH_2COOCH_2(1\text{-}C_7H_{15})$ | " | 0 | oil | 4.08m* ($CDCl_3$) |
| 20 | " | " | $CH_2CH_2OOCNHCH_3$ | " | 0 | v.oil | 4.31t* ($CDCl_3$) |
| 21 | " | " | $CH_2CH_2N(CH_2CH_3)_2$ | " | 0 | wax | 1.01m* ($CDCl_3$) |
| 22 | " | $CH_3$ | see (1) below | " | 0 | oil | 2.95s* ($CDCl_3$) |
| 23 | " | $CH_3$ | $CH_2C_6H_5$ | " | 0 | 136.5-138.5 | 3.89m*($CDCl_3$) |
| 24 | $C_6H_5$ | $CH_3$ | $CH_2C_6H_4\text{-}4\text{-}(OCH_3)$ | $2\text{-}F\text{-}C_6H_4$ | 0 | 113-115.5 | 3.81m* ($CDCl_3$) |
| 25 | " | $CH_3$ | $CH(CH_3)C_6H_5$ | " | 0 | 90-93.5 | 2.80s ($CDCl_3$) |
| 26 | " | $CH_3$ | $CH_2CH_2C_6H_5$ | " | 0 | 95-97 | 2.84s* ($CDCl_3$) |
| 27 | " | $CH_2C{\equiv}CH$ | $CH_3$ | " | 0 | oil | 4.01m ($CDCl_3$) |
| 28 | $2\text{-}OH\text{-}C_6H_4$ | $CH_3$ | " | " | 0 | 68 | 8.74s* ($CDCl_3$) |
| 29 | $3,4\text{-}Cl_2\text{-}C_6H_3$ | " | $CH_3$ | " | 0 | 131-132 | 2.09s ($CDCl_3$) |
| 30 | $4\text{-}CH_3\text{-}C_6H_5$ | " | $CH_3$ | " | 0 | 108-110 | 2.28s ($CDCl_3$) |
| 31 | $4\text{-}CH_3O\text{-}C_6H_4$ | " | " | " | 0 | 79.5-82 | 3.78s*($CDCl_3$) |

| Compound B-No. | R | R¹ | R² | R³ | X | m.p. °C | δ (Solvent) |
|---|---|---|---|---|---|---|---|
| 32 | $4\text{-}CH_3O\text{-}C_6H_4$ | $CH_3$ | $CH_2C_6H_5$ | $2\text{-}F\text{-}C_6H_4$ | 0 | 86-88.5 | 2.73s* (CDCl$_3$) |
| 33 | " | $CH_3$ | $CH_2CH_2COOCH_3$ | " | 0 | 72.5-75 | 2.92s* (CDCl$_3$) |
| 34 | " | $CH_3$ | $CH_2CH_2COO\text{-}i\text{-}C_8H_{17}$ | " | 0 | oil | 2.93s* (CDCl$_3$) |
| 35 | $2,5\text{-}(CH_3O)_2\text{-}C_6H_3$ | $CH_3$ | $CH_3$ | " | 0 | 89-90 | 6.5s (CDCl$_3$) |
| 36 | $3,4\text{-}(CH_3O)_2\text{-}C_6H_3$ | " | $CH_3$ | " | 0 | 125.5-127 | 2.17s* (CDCl$_3$) |
| 37 | " | " | $CH_2CH_2OC_2H_5$ | " | 0 | 65.5-68 | 2.8m (CDCl$_3$) |
| 38 | " | " | $CH_2CH_2COOCH_3$ | " | 0 | wax | 3.68s* (CDCl$_3$) |
| 39 | " | $CH_3$ | $CH_2CH_2COO\text{-}i\text{-}C_8H_{17}$ | " | 0 | oil | 2.93s* (CDCl$_3$) |
| 40 | " | $CH_3$ | $CH_2CH_2N(CH_2CH_3)_2$ | " | 0 | oil | 1.02m* (CDCl$_3$) |
| 41 | $4\text{-}CH_3\text{-}C_6H_4$ | " | furfuryl | " | 0 | 94-96 | 2.28s (CDCl$_3$) |
| 42 | $3,4\text{-}(CH_3O)_2\text{-}C_6H_3$ | $n\text{-}C_4H_9$ | $CH_3$ | " | 0 | oil | 2.11s (CDCl$_3$) |
| 43 | " | $CH_2CH{=}CH_2$ | $CH_3$ | " | 0 | oil | 3.95m (CDCl$_3$) |
| 44 | " | $c\text{-}C_6H_{11}$ | $CH_3$ | " | 0 | oil | 2.27s (CDCl$_3$) |
| 45 | " | $CH_2\text{-}C_6H_5$ | $CH_3$ | " | 0 | 123-124 | 2.15s (CDCl$_3$) |
| 46 | $3,4\text{-}(CH_2O_2)\text{-}C_6H_3$ | $CH_3$ | $CH_3$ | " | 0 | 88-92 | 5.93s* (CDCl$_3$) |

Table III - continued

| Compound B-No. | R | R¹ | R² | R³ | X | m.p. °C | δ (Solvent) |
|---|---|---|---|---|---|---|---|
| 47 | $3,4\text{-}(CH_2O_2)\text{-}C_6H_3$ | $CH_3$ | $CH_2CH_2COOCH_3$ | $2\text{-F-}C_6H_4$ | 0 | oil | 5.92s* (CDCl$_3$) |
| 48 | $3\text{-}C_6H_5O\text{-}C_6H_4$ | " | $CH_3$ | " | 0 | 102-107 | 2.10s (CDCl$_3$) |
| 49 | $(CH_3)_2NC_6H_4$ | " | $CH_3$ | " | 0 | 110.5-113.5 | 2.97s* (CDCl$_3$) |
| 50 | 2-pyrryl | $CH_3$ | $CH_2CH_2COO\text{-}n\text{-}C_4H_9$ | " | 0 | oil | 2.83s (CDCl$_3$) |
| 51 | 2-pyridyl | $CH_3$ | $CH_3$ | " | 0 | dec. | 6.87s* (CDCl$_3$) |
| 52 | 2-pyridyl | " | $CH_2CH_2COO\text{-}n\text{-}C_{12}H_{25}$ | " | 0 | v.oil | 7.01s* (CDCl$_3$) |
| 53 | 3-pyridyl | " | $CH_3$ | " | 0 | 79 | 2.19s* (CDCl$_3$) |
| 54 | " | " | $CH_2COOCH_3$ | " | 0 | wax | 3.48s* (CDCl$_3$) |
| 55 | " | " | $CH_2CH_2COOCH_3$ | " | 0 | oil | 7.10s* (CDCl$_3$) |
| 56 | 4-pyridyl | " | $CH_3$ | " | 0 | 134-136 | 2.19s* (CDCl$_3$) |
| 57 | " | " | $CH_2CH_2OH$ | " | 0 | oil | 3.86m* (CDCl$_3$) |
| 58 | " | " | $CH_2COOCH_3$ | " | 0 | oil | 3.50s* (CDCl$_3$) |
| 59 | " | " | $CH_2CH_2COOCH_3$ | " | 0 | 81 | 3.69s* (CDCl$_3$) |
| 60 | 2-furyl | " | $CH_3$ | " | 0 | 104-105 | 2.17s* (CDCl$_3$) |
| 61 | " | " | $CH_2CH_2COOCH_3$ | " | 0 | 85 | 3.66s* (CDCl$_3$) |
| 62 | 2-thienyl | " | $CH_3$ | " | 0 | 124.5-128 | 2.19s* (CDCl$_3$) |
| 63 | " | " | $CH_2COOCH_3$ | " | 0 | wax | 3.68s* (CDCl$_3$) |

-42-

0093610

Table III - continued

| Compound B- No. | R | R$^1$ | R$^2$ | R$^3$ | X | m.p. °C | δ (Solvent) |
|---|---|---|---|---|---|---|---|
| 64 | 2-thienyl | $CH_3$ | $CH_2CH_2COOCH_3$ | $2\text{-}F\text{-}C_6H_4$ | 0 | 87 | 3.01s* ($CDCl_3$) |
| 65 | " | $CH_3$ | $CH_2CH_2N(CH_2CH_3)_2$ | " | 0 | oil | 0.98m* ($CD_3COCD_3$) |
| 66 | 3-indolyl | $CH_3$ | $CH_3$ | " | 0 | v. oil | 2.78s ($CDCl_3$) |
| 68 | $4\text{-}n\text{-}C_5H_{11}O\text{-}C_6H_4$ | " | " | $2,5\text{-}F_2\text{-}C_6H_3$ | 0 | 91-93.5 | 2.90s ($CDCl_3$) |
| 69 | $C_6H_5$ | $CH_3$ | $CH_2CH_2COOCH_3$ | $4\text{-}CH_3S\text{-}C_6H_4$ | 0 | wax | 2.43s ($CDCl_3$) |
| 70 | $3,4\text{-}(CH_3O)_2\text{-}C_6H_3$ | " | $CH(CH_3)CH=CH_2$ | $2\text{-}F\text{-}C_6H_4$ | 0 | oil | 4.83m ($CDCl_3$) |

Remarks:

(1)  $CH(C_2H_5)CH_2\text{-}(2,6\text{-}(CH_3)_2\text{-}4\text{-morpholinyl})$

## Table IV

### Group "C" Compounds

Structural Formula: $R^2SCH(R)N(R^1)C(O)NHR^3$

| Compound C-No. | R | $R^1$ | $R^2$ | $R^3$ | m.p. °C | δ (Solvent) |
|---|---|---|---|---|---|---|
| 1 | $C_6H_5$ | $CH_3$ | $4-OH-C_6H_4$ | $2-F-C_6H_4$ | 153-154.5 | 7.01s* ($CD_3SOCD_3$) |
| 2 | " | " | $4-CH_3-C_6H_4$ | " | 132.5-135 | 2.26s* ($CDCl_3$) |
| 3 | " | " | $2-CH_3O-C_6H_4$ | " | 106-110.5 | 3.88s* ($CDCl_3$) |
| 4 | " | " | $3-CH_3O-C_6H_4$ | " | 112.5-114.5 | 3.73s* ($CDCl_3$) |
| 5 | " | " | $4-CH_3O-C_6H_4$ | " | 124-127.5 | 3.72s* ($CDCl_3$) |
| 6 | $2-OH-C_6H_4$ | $CH_3$ | $4-OH-C_6H_4$ | " | 131 | 2.84s* ($CD_3SOCD_3$) |
| 7 | $4-OH-C_6H_4$ | $CH_3$ | $4-CH_3O-C_6H_4$ | " | 117.5-120 | 3.65s ($CD_3COCD_3$) |
| 8 | $4-CH_3O-C_6H_4$ | " | $4-OH-C_6H_4$ | " | 158-160.5 | 3.76s* ($CD_3SOCD_3$) |
| 9 | $4-CH_3O-C_6H_4$ | " | $4-CH_3-C_6H_4$ | " | 144.5-145.5 | 2.26s* ($CDCl_3$) |
| 10 | $3,4-(CH_3O)_2-C_6H_3$ | " | $4-OH-C_6H_4$ | " | 159.5-162 | 3.76* ($CD_3SOCD_3$) |
| 11 | $3,4-(CH_3O)_2-C_6H_3$ | $CH_3$ | $4-CH_3O-C_6H_4$ | " | 115-117.5 | 2.93s* ($CDCl_3$) |
| 13 | $3,4-(CH_2O_2)-C_6H_3$ | $CH_3$ | $4-OH-C_6H_4$ | " | 174.5-176 | 6.04s* ($CD_3SOCD_3$) |
| 14 | $4-(CH_3)_2N-C_6H_4$ | " | $4-OH-C_6H_4$ | " | 173-175 | 9.58s* ($CD_3SOCD_3$) |
| 15 | 4-pyridyl | $CH_3$ | $4-OH-C_6H_4$ | " | 141.5-144 | 3.10s* ($CD_3COCD_3$) |
| 16 | 2-furyl | $CH_3$ | $4-OH-C_6H_4$ | " | 135 | 8.56s* ($CD_3COCD_3$) |
| 17 | 2-thienyl | " | $4-OH-C_6H_4$ | " | 109.5-111.5 | 9.68s* ($CD_3SOCD_3$) |

-44-

Table IV - continued

| Compound C- No. | R | R$^1$ | R$^2$ | R$^3$ | m.p. $^oC$ | $\delta$ (Solvent) |
|---|---|---|---|---|---|---|
| 18 | 4-C$\underline{H}_3$-C$_6$H$_4$ | CH$_3$ | 4-CH$_3$O-C$_6$H$_4$ | 2,4-F$_2$-C$_6$H$_3$ | 116-120 | 2.30s (CDCl$_3$) |
| 19 | 4-n-C$_5$H$_{11}$O-C$_6$H$_4$ | " | 4-CH$_3$O-C$_6$H$_4$ | 2,5-F$_2$-C$_6$H$_3$ | 102-103.5 | 3.63s (CDCl$_3$) |
| 20 | 3,4-(CH$_3$O)$_2$-C$_6$H$_3$ | CH$_2$CH=CH$_2$ | 4-C$\underline{H}_3$O-C$_6$H$_4$ | " | wax | 3.69 (CDCl$_3$) |
| 21 | " | CH$_3$ | 4-O$\underline{H}$-C$_6$H$_4$ | 2,6-F$_2$-C$_6$H$_3$ | 179-181.5 | 9.50s (CD$_3$SOCD$_3$) |
| 22 | C$_6$H$_5$ | " | 4-CH$_3$O-C$_6$H$_4$ | 3-CF$_3$-C$_6$H$_4$ | oil | 3.82s* (CDCl$_3$) |
| 23 | " | " | 4-O$\underline{H}$-C$_6$H$_4$ | C$_6$H$_5$ | 167-169 | 9.59s* (CD$_3$SOCD$_3$) |
| 24 | 3,4-(C$\underline{H}_3$O)$_2$-C$_6$H$_3$ | " | 4-OH-C$_6$H$_4$ | " | 155-159 | 3.76* (CD$_3$SOCD$_3$) |
| 25 | C$_6$H$_5$ | CH$_2$C≡CH | 4-CH$_3$O-C$_6$H$_4$ | 2-F-C$_6$H$_4$ | 96-108 | 3.04s (CD$_3$COCD$_3$) |

The compounds of this invention are very effective herbicides, either for preemergence and/or postemergence application, i.e., for control of undesirable vegetation. The control of weeds is of great economic importance since weed competition inhibits the production of foliage, fruit, or seed of agricultural crops. The presence of weeds can reduce harvesting efficiency and the quality of the harvested crop. Weed control is essential for maximum production of many agronomic and horticultural crops including corn, (Zea mays L.), barley (Hordeum vulgare L.) and oats (Avena sativa L.) and soybeans (Glycine max (L.) Merr.). Weeds on noncropped areas may cause a fire hazard, undesirable drifting of sand or snow, or irritation to persons with allergies. Thus, suppression of undesirable weed growth would have many advantages.

It has been found that the substituted ureas of this invention are remarkably effective as preemergence and postemergence herbicides.

To use the present chemicals as herbicides, the chemicals are applied, in a herbicidally effective amount, to a locus where such control is to be effected, i.e. either to the plant itself and/or to the soil in which the plant is growing or is to be grown. The chemical is suitably applied as a formulation in accordance with conventional agricultural chemical practice.

Thus, the chemical may be impregnated on finely-divided or granular inorganic or organic carriers such as attapulgite clay, sand, vermiculite, ground corn cobs, activated carbon or other granular carriers known to the art. The impregnated granules may then be spread on the soil. Furthermore, the chemical may be formulated, for example, as a wettable powder by grinding it into a fine powder and mixing it with an inactive powdered carrier to which a surface active dispersing agent has been added. Typical powdered solid carriers are the various mineral silicates, e.g., mica, talc, pyrophyllite, and clays. The wettable powder may then be dispersed in water and sprayed on weeds or onto the soil surface. Similarly, an emulsifiable concentrate may be prepared by dissolving the chemical in a solvent such as benzene, toluene, or other aliphatic or aromatic hydrocarbon to which a surface active and/or dispersing agent(s) has been added. The emulsifiable concentrate may then be dispersed in water and applied by spraying. Water solubility may be increased using a cosolvent system involving acetone, dimethyl sulfoxide or other water miscible solvents. Suitable surface active agents are well known to those skilled in the art, and reference may be had to McCutcheon's Detergents and Emulsifiers, 1970, Allured Publishing Corp., Ridgewood, New Jersey, or Hoffman et al, U.S. Patents 2,614,916, cols 2 to 4 and 2,547,724, cols. 3 and 4.

The concentration of an active chemical in the formulation may vary widely, e.g. from 1 to 95%. The concentration of active chemical in dispersions applied to the soil or foliage is almost invariably from 0.002% to 75%.

For use as preemergence herbicides the chemicals are frequently applied at rates of 0.05 to 25 pounds per acre (0.056 to 28 kg/ha) to soil which contains weed and crop seed, namely either to the surface of the soil or incorporated into the upper one to three inches (2.5 to 7.5 cm.) of soil. As postemergence herbicides, the chemicals are typically applied at rates of 0.05 to 25 pounds per acre (0.056 to 28 kg/ha) to the foliage of weeds. The chemicals may be employed singly or as a mixture of two or more chemicals.

The most suitable rate of application in any given case may depend on such factors as soil type, soil pH, soil organic matter content, the quantity and intensity of rainfall before and after treatment, the air and soil temperature, light intensity and light duration per day. All of these factors can have an influence on the efficacy of the chemicals for a given weed control use.

The herbicidal use may include control of vegetation at industrial sites or selective weed control in crops.

## Example 19

To illustrate effectiveness of the previously described substituted ureas of this invention as preemergence herbicides, 600 mg chemical is dissolved in 10 ml organic solvent (e.g. acetone) to which 30 mg emulsifying agent "Triton [trademark] X100" (isooctyl-polyethoxyethanol) is added. The solution is diluted to 100 ml with distilled water. Ten milliliters of this 6000 ppm solution is diluted to 250 ppm with distilled water. The chemical is applied at the rate of 10 1b/A (11.2 kg/ha) by drenching 46 ml of the 250 ppm solution on the surface of soil in 4-1/2 inch (11.25 cm) plastic pots wherein seeds of the following weeds had been planted: rough pigweed ( Amaranthus retroflexus L.) or velvetleaf ( Abutilon theophrasti medic) jimsonweed ( Datura stramonium L.), tall morningglory ( Ipomea • purpurea L. Roth), crabgrass ( Digitaria ischaemum [Schreb.] Muhl.), or switchgrass ( Panicum virgatum L.), barnyardgrass ( Echinochloa crusgalli (L.) Beauv.), green foxtail ( Setaria viridis (L.), Beauv.). The percent control of the weeds compared to untreated checks is determined two weeks after treatment. TABLES V to VII summarize the results achieved with compounds formulated as indicated above, and the data clearly indicate the good to excellent herbicidal efficacy of compounds of this invention.

                              Example 20

To illustrate effectiveness of the described substituted ureas as postemergence herbicides, the 6000 ppm solution described under Example 19 is atomized with a conventional DeVilbiss [trademark] sprayer, wetting the foliage to the drip point. The weeds, which are the same species as described under Example 19 are treated six days after emergence. The percent weed control is evaluated two weeks after treatment. Tables VIII to X illustrate the postemergence herbicidal efficacy of chemicals of this invention.

Tables V - X seem to indicate that Group A chemicals generally have pre- and postemergence activity, Group B compounds excel as postemergence herbicides and Group C chemicals provide good pre- and/or postemergence efficacy.

-49-

Table V

Group "A" Compounds

Preemergence Weed Control, %

(at 10 lb/A (11.2 kg/ha))

| Compound A- No. | Velvetleaf or Pigweed* | Jimson Weed | Morning Glory | Barnyard Grass | Switchgrass | Green Foxtail |
|---|---|---|---|---|---|---|
| 1 | 50 | 0 | 25 | 40 | 0 | 10 |
| 2 | 100 | 50 | 100 | 100 | 90 | 100 |
| 4 | 100 | 40 | 100 | 95 | 70 | 95 |
| 5 | 100* | 0 | 100 | 95 | 95 | 95 |
| 6 | 100 | 50 | 75 | 95 | 25 | 75 |
| 7 | 100 | 0 | 100 | 95 | 35 | 95 |
| 8 | 50* | 0 | 100 | 100 | 50 | 100 |
| 9 | 100 | 20 | 100 | 100 | 50 | 95 |
| 10 | 100 | 30 | 90 | 95 | 65 | 90 |
| 11 | 90 | 0 | 25 | 35 | 0 | 0 |
| 12 | 100 | 100 | 50 | 100 | 80 | 100 |
| 13 | 100 | 0 | 35 | 95 | 0 | 95 |
| 14 | 90 | 0 | 50 | 90 | 75 | 95 |
| 15 | 100 | 50 | 80 | 100 | 35 | 95 |
| 16 | 100 | 0 | 0 | 0 | 0 | 0 |
| 17 | 100 | 80 | 100 | 100 | 50 | 100 |
| 18 | 100 | 90 | 100 | 100 | 75 | 100 |
| 19 | 100 | 0 | 15 | 85 | 25 | 75 |
| 20 | 100 | 95 | 100 | 100 | 100 | 100 |
| 22 | 100 | 0 | 90 | 100 | 75 | 100 |
| 23 | 100 | 70 | 100 | 95 | 70 | 90 |
| 24 | 100 | 60 | 85 | 100 | 90 | 95 |
| 25 | 100 | 0 | 100 | 100 | 5 | 100 |
| 26 | 100 | 0 | 80 | 100 | 80 | 100 |
| 27 | 100 | 0 | 0 | 95 | 80 | 95 |
| 28 | 100 | 30 | 30 | 70 | 20 | 40 |
| 29 | 100 | 0 | 95 | 80 | 100 | 75 |
| 30 | 100 | 0 | 70 | 100 | 80 | 95 |
| 31 | 80 | 20 | 0 | 20 | 0 | 30 |
| 32 | 100* | 0 | 0 | 35 | 0 | 75 |
| 33 | 100* | 0 | 0 | 100 | 100 | 100 |
| 34 | 100* | 0 | 75 | 95 | 95 | 100 |
| 35 | 100* | 0 | 100 | 95 | 75 | 95 |

Table V (continued)

| Compound A-No. | Velvetleaf or Pigweed* | Jimson Weed | Morning Glory | Barnyard Grass | Switchgrass | Green Foxtail |
|---|---|---|---|---|---|---|
| 36 | 100 | 20 | 100 | 90 | 50 | 90 |
| 38 | 100 | 30 | 100 | 85 | 30 | 80 |
| 39 | - | 0 | 0 | 75 | 50 | 50 |
| 40 | 0* | 0 | 50 | 25 | 25 | 25 |
| 42 | 100 | 0 | 100 | 100 | 75 | 95 |
| 43 | 100 | 10 | 100 | 100 | 0 | 100 |
| 44 | 100 | 0 | 80 | 85 | 25 | 95 |
| 45 | 95 | 40 | 100 | 95 | 30 | 90 |
| 47 | 100* | 0 | 0 | 100 | 50 | 90 |
| 48 | 100 | 25 | 85 | 100 | 0 | 50 |
| 49 | 90 | 0 | 100 | 90 | 30 | 80 |
| 50 | 100 | 25 | 100 | 100 | 100 | 100 |
| 51 | 100 | 0 | 90 | 100 | 75 | 100 |
| 54 | 50 | 0 | 0 | 90 | 75 | 75 |
| 55 | 100 | 50 | 75 | 90 | 30 | 90 |
| 56 | 100 | 35 | 50 | 100 | 65 | 90 |
| 57 | 100 | 0 | 15 | 100 | 50 | 95 |
| 58 | 0 | 30 | 0 | 50 | 0 | 50 |
| 59 | 50 | 0 | 25 | 0 | 0 | 50 |
| 60 | 0 | 0 | 0 | 50 | 5 | 25 |
| 61 | 0 | 0 | 0 | 50 | 20 | 50 |
| 62 | 80 | 0 | 60 | 50 | 25 | 90 |
| 63 | 100 | 0 | 35 | 40 | 15 | 75 |

-51-

Table VI

Group "B" Compounds

Preemergence Weed Control, %
(at 10 lbs/A (11.2 kg/ha))

| Compound B-No. | Velvetleaf or Pigweed* | Jimson Weed | Morning Glory | Barnyard Grass | Crabgrass** or Switchgrass | Green Foxtail |
|---|---|---|---|---|---|---|
| 3 | 0* | 0 | 0 | 20 | 25** | 25 |
| 4 | 100* | 0 | 0 | 50 | 80** | 50 |
| 5 | 100* | 0 | 0 | 100 | 100 | 100 |
| 7 | 40* | 0 | 0 | 0 | 0** | 0 |
| 8 | 0 | 0 | 0 | 75 | 50 | 45 |
| 9 | 100* | 0 | 95 | 100 | 100** | 100 |
| 13 | 100* | 100 | 0 | 100 | 100** | 100 |
| 15 | - | 0 | 80 | 100 | 95 | 95 |
| 16 | 100* | 0 | 100 | 95 | 100 | 100 |
| 17 | 0* | 0 | 0 | 30 | 30** | 30 |
| 18 | 100* | 25 | 100 | 98 | 100** | 100 |
| 19 | 90* | 0 | 0 | 30 | 30** | 30 |
| 20 | 100* | 0 | 80 | 50 | 60 | 90 |
| 21 | 0* | 0 | 0 | 100 | 100** | 100 |
| 22 | - | 0 | 0 | 80 | 75 | 80 |
| 25 | 100* | 5 | 75 | 100 | 90 | 75 |
| 26 | 75* | 0 | 0 | 0 | 0 | 0 |
| 28 | - | 0 | 0 | 95 | 75 | 80 |
| 30 | 100 | 0 | 100 | 100 | 65 | 100 |
| 31 | 100* | 0 | 100 | 100 | 100** | 100 |
| 33 | 100* | 0 | 100 | 95 | 100** | 100 |
| 34 | 100* | 95 | 100 | 100 | 100** | 100 |
| 35 | 100 | 0 | 100 | 95 | 0 | 75 |
| 36 | 100* | 0 | 100 | 100 | 100** | 100 |
| 37 | 100 | 0 | 80 | 100 | 25 | 75 |
| 38 | 100* | 0 | 100 | 100 | 95** | 95 |
| 39 | 75* | 0 | 50 | 95 | 95** | 95 |
| 40 | - | 50 | 75 | 100 | 100 | 100 |

-52-

Table VI, continued

| Compound B- No. | Velvetleaf or Pigweed* | Jimson Weed | Morning Glory | Barnyard Grass | Crabgrass* * or Switchgrass | Gree Foxta |
|---|---|---|---|---|---|---|
| 42 | 100 | 0 | 75 | 95 | 75 | 100 |
| 43 | 100 | 0 | 50 | 90 | 75 | 90 |
| 46 | 100* | 0 | 100 | 100 | 0 | 100 |
| 47 | - | 0 | 100 | 75 | 75 | 75 |
| 49 | 0* | 0 | 90 | 100 | 100 | 100 |
| 50 | 100 | 65 | 100 | 95 | 50 | 90 |
| 51 | - | 0 | 80 | 100 | 50 | 100 |
| 53 | 100* | 80 | 100 | 100 | 100 | 100 |
| 54 | 75* | 0 | 0 | 75 | 25 | 90 |
| 55 | 50* | 0 | 100 | 100 | 100 | 100 |
| 57 | 0* | 0 | 0 | 30 | 50 | 80 |
| 59 | 0* | 0 | 90 | 10 | 0 | 25 |
| 60 | - | - | 100 | 100 | 95 | 100 |
| 61 | - | 0 | 100 | 85 | 100 | 100 |
| 62 | - | 0 | 90 | 100 | 80 | 100 |
| 63 | 100* | 0 | 90 | 90 | 90 | 90 |
| 64 | - | 0 | 90 | 65 | 50 | 25 |
| 65 | 0* | 0 | 0 | 100 | 100 | 100 |

## Table VII

### Group "C" Compounds

### Preemergence Weed Control, %

### (at 10 lbs/A (11.2 kg/ha))

| Compound C-No. | Velvetleaf or Pigweed* | Jimson Weed | Morning Glory | Barnyard Grass | Crabgrass** or Switchgrass | Green Foxtail |
|---|---|---|---|---|---|---|
| 1 | 100* | 40 | 95 | 40 | 35** | 60 |
| 3 | 100* | 0 | 0 | 0 | 0** | 0 |
| 5 | 100* | 0 | 100 | 95 | 95** | 98 |
| 6 | 0* | 0 | 90 | 0 | 0 | 0 |
| 7 | 100 | 0 | 35 | 90 | 95 | 95 |
| 8 | - | 0 | 75 | 75 | 90** | 90 |
| 10 | 100* | 0 | 90 | 95 | 90** | 90 |
| 14 | 0* | 0 | 0 | 0 | 0 | 75* |
| 16 | - | 0 | 100 | 100 | 100 | 100 |
| 17 | - | 0 | 90 | 100 | 90 | 100 |
| 20 | 100 | 0 | 0 | 75 | 50 | 90 |
| 23 | 95 | 0 | 95 | 25 | - | 25 |
| 24 | 50 | 0 | 50 | 80 | 80 | 90 |

-54-

## Table VIII
### Group "A" Compounds
### Postemergence Weed Control, %
(at 6000 ppm)

| Compound A-No. | Velvetleaf or Pigweed* | Jimson Weed | Morning Glory | Barnyard Grass | Switchgrass | Green Foxtail |
|---|---|---|---|---|---|---|
| 1 | 95 | 0 | 95 | 15 | 0 | 5 |
| 2 | 100 | 95 | 100 | 100 | 90 | 100 |
| 3 | 100 | 20 | 100 | 60 | 50 | 70 |
| 4 | 100 | 100 | 100 | 100 | 100 | 100 |
| 5 | - | 100 | 100 | 100 | 100 | 100 |
| 6 | 100 | 0 | 100 | 75 | 0 | 75 |
| 7 | 100 | 0 | 100 | 95 | 100 | 100 |
| 8 | 100* | 100 | 100 | 100 | - | 100 |
| 9 | 100 | 100 | 100 | 100 | 90 | 95 |
| 10 | 100 | 95 | 100 | 95 | 75 | 100 |
| 11 | 100 | 0 | 100 | 100 | 65 | 80 |
| 12 | 100 | 0 | 95 | 80 | 75 | 90 |
| 13 | 100 | 80 | 100 | 100 | 50 | 100 |
| 14 | 100 | 0 | 100 | 100 | 100 | 100 |
| 15 | 100 | 0 | 100 | 85 | 80 | 95 |
| 16 | - | 100 | 100 | 100 | 75 | 100 |
| 17 | 100 | 75 | 100 | 100 | 100 | 100 |
| 18 | 100 | 25 | 100 | 95 | 80 | 100 |
| 19 | 100 | 5 | 100 | 100 | 100 | 100 |
| 20 | 100 | 95 | 100 | 100 | 100 | 100 |
| 22 | 100 | 5 | 100 | 100 | 50 | 100 |
| 23 | 100 | 90 | 100 | 95 | 35 | 90 |
| 24 | 100 | 25 | 100 | 80 | 10 | 65 |
| 25 | 100 | 100 | 100 | 100 | 80 | 75 |
| 26 | 100 | 95 | 100 | 100 | 90 | 100 |
| 27 | 100 | 0 | 100 | 100 | 90 | 100 |
| 28 | 100 | 95 | 100 | 100 | 100 | 100 |
| 29 | 100 | 25 | 100 | 100 | 90 | 100 |
| 30 | 100 | 25 | 100 | 100 | 100 | 100 |
| 31 | 100 | 60 | 100 | 100 | 25 | 95 |
| 32 | 100* | 50 | 100 | 100 | 100 | 100 |
| 33 | 100* | 50 | 90 | 90 | 90 | 95 |
| 34 | - | 100 | 100 | 100 | 100 | 100 |

-55-

Table VIII, continued

| Compound A- No. | Velvetleaf or Pigweed* | Jimson Weed | Morning Glory | Barnyard Grass | Switchgrass | Green Foxtail |
|---|---|---|---|---|---|---|
| 36 | 100 | 5 | 100 | 95 | 30 | 75 |
| 38 | 100 | 85 | 100 | 100 | - | - |
| 39 | - | 75 | 100 | 100 | 100 | 100 |
| 40 | 50* | 0 | 5 | 5 | 5 | 5 |
| 42 | 100 | 65 | 100 | 100 | 100 | 100 |
| 43 | 100 | 70 | 95 | 100 | 95 | 100 |
| 44 | 100 | 100 | 100 | 100 | 50 | 100 |
| 45 | 90 | 60 | 100 | 95 | 20 | 80 |
| 47 | 100* | 100 | 100 | 100 | 100 | 100 |
| 48 | 100 | 50 | 100 | 100 | 100 | 100 |
| 49 | 100 | 95 | 100 | 100 | - | - |
| 50 | 100 | 80 | 100 | 20 | 35 | 25 |
| 51 | 100 | 50 | 100 | 100 | 100 | 100 |
| 52 | 0 | 0 | 40 | 10 | 0 | 0 |
| 53 | 0 | 0 | 0 | 10 | 0 | 5 |
| 54 | 100 | 25 | 100 | 90 | 15 | 50 |
| 55 | 100 | 95 | 100 | 100 | - | - |
| 56 | 100 | 85 | 100 | 100 | 90 | 100 |
| 57 | 100 | 30 | 100 | 95 | 80 | 100 |
| 58 | 100 | 95 | 95 | 100 | - | - |
| 59 | 95 | 80 | 100 | 65 | 100 | 100 |
| 60 | 100 | 0 | 100 | 50 | 25 | 80 |
| 61 | 100 | 5 | 100 | 95 | 90 | 90 |
| 62 | 100 | 100 | 100 | 80 | 15 | 75 |
| 63 | 25 | 50 | 100 | 25 | 0 | 15 |
| 64 | 25 | 0 | 100 | 75 | 100 | 100 |
| 65 | 0 | 0 | 95 | 85 | 0 | 50 |
| 66 | 0 | 0 | 25 | 5 | 0 | 5 |
| 67 | 30 | 15 | 80 | 55 | 10 | 75 |

-56-

## Table IX
### Group "B" Compounds
#### Postemergence Weed Control, %
(at 6000 ppm)

| Compound B- No. | Velvetleaf or Pigweed* | Jimson Weed | Morning Glory | Barnyard Grass | Crabgrass** or Switchgrass | Green Foxtail |
|---|---|---|---|---|---|---|
| 1 | 100* | 0 | 90 | 10 | 0** | 25 |
| 2 | - | 25 | 15 | 0 | 100 | 100 |
| 3 | 100* | 20 | 95 | 75 | 85** | 35 |
| 4 | 100* | 100 | 100 | 100 | 100** | 100 |
| 5 | 85* | 75 | 95 | 20 | 100 | 100 |
| 6 | 0* | 0 | 50 | 25 | 50 | 75 |
| 7 | 100 | 0 | 75 | 40 | 90** | 95 |
| 8 | 100 | 0 | 100 | 100 | 100 | 100 |
| 9 | 80* | 0 | 5 | 10 | 5** | 5 |
| 10 | 100* | 50 | 100 | 45 | 75** | 75 |
| 11 | 100* | 100 | 100 | 100 | 100** | 95 |
| 12 | 100* | 90 | 100 | 25 | 30** | 30 |
| 13 | 100* | 100 | 100 | 95 | 100** | 100 |
| 14 | 100* | 0 | 95 | 10 | 0** | 0 |
| 15 | - | 100 | 100 | 100 | 100 | 100 |
| 16 | 100* | 100 | 100 | 100 | 100** | 100 |
| 17 | 100* | 100 | 100 | 95 | 100** | 100 |
| 18 | 100* | 0 | 100 | 100 | 100** | 100 |
| 19 | 100* | 100 | 100 | 100 | 100** | 100 |
| 20 | 0* | 100 | 100 | 100 | 100 | 100 |
| 21 | 100* | 90 | 95 | 100 | 100** | 100 |
| 22 | - | 100 | 100 | 100 | 100 | 100 |
| 23 | 15* | 0 | 80 | 5 | 5** | 10 |
| 24 | 100* | 0 | 100 | 50 | 95** | 90 |
| 25 | 100* | 75 | 100 | 65 | 30 | 75 |
| 26 | 90* | 100 | 100 | 100 | 100** | 100 |
| 27 | 100 | 25 | 100 | 40 | 60 | 90 |
| 28 | - | - | 25 | 100 | 75 | 95 |
| 29 | 0 | 0 | 65 | 0 | 0 | 0 |
| 30 | 100 | 0 | 100 | 100 | 100 | 100 |
| 31 | 100* | 100 | 100 | 100 | 100** | 100 |
| 32 | 100* | 0 | 100 | 50 | 5** | 30 |
| 33 | 100* | 100 | 100 | 100 | 100** | 100 |

-57-
Table IX, continued

| Compound B- No. | Velvetleaf or Pigweed* | Jimson Weed | Morning Glory | Barnyard Grass | Crabgrass** or Switchgrass | Green Foxtail |
|---|---|---|---|---|---|---|
| 34 | 100* | 100 | 100 | 100 | 100** | 100 |
| 35 | 100 | 70 | 100 | 100 | 100 | 100 |
| 36 | 100* | 100 | 100 | 100 | 100 | 100 |
| 37 | 100 | 50 | 100 | 100 | 50 | 100 |
| 38 | 100* | 100 | 100 | 100 | 100** | 100 |
| 39 | 100* | 100 | 100 | 100 | 100** | 100 |
| 40 | 100* | 100 | 100 | 100 | 100 | 100 |
| 42 | 100 | 15 | 100 | 100 | 100 | 100 |
| 43 | 100 | 0 | 95 | 80 | 100 | 100 |
| 44 | 55 | 0 | 100 | 60 | 90 | 100 |
| 45 | 0 | 0 | 0 | 0 | 0 | 10 |
| 46 | - | 100 | 100 | 100 | 100 | 100 |
| 47 | - | 90 | 100 | 100 | 100 | 100 |
| 48 | 90 | 0 | 95 | 10 | 0 | 0 |
| 49 | 100* | 0 | 100 | 100 | 75 | 100 |
| 50 | 100 | 95 | 100 | 75 | 45 | 90 |
| 51 | - | - | 100 | 98 | - | 100 |
| 52 | - | - | 100 | 85 | 95 | 100 |
| 53 | 100* | 100 | 100 | 90 | 100 | 100 |
| 54 | 100* | 100 | 100 | 55 | 100 | 100 |
| 55 | 100* | 100 | 100 | 50 | 100 | 100 |
| 56 | - | 90 | 100 | 60 | 75 | 25 |
| 57 | - | 90 | 100 | 100 | 100 | 100 |
| 58 | - | 100 | 100 | 80 | 75 | 100 |
| 59 | 100* | 95 | 100 | 100 | 100 | 100 |
| 60 | 100* | 100 | 100 | 100 | 100 | 100 |
| 61 | 100* | 100 | 100 | 100 | 100 | 100 |
| 62 | - | 100 | 100 | 100 | 100 | 100 |
| 63 | 100* | 100 | 100 | 100 | 100 | 100 |
| 64 | - | 95 | 100 | 100 | 100 | 100 |
| 65 | - | 100 | 100 | 100 | - | 100 |
| 69 | 100* | 15 | 100 | 100 | 100** | 65 |

-58-

## Table X

### Group "C" Compounds
#### Postemergence Weed Control, %
(at 6000 ppm)

| Compound C-No. | Velvetleaf or Pigweed* | Jimson Weed | Morning Glory | Barnyard Grass | Crabgrass** or Switchgrass | Green Foxtail |
|---|---|---|---|---|---|---|
| 1 | 0. | 0 | 10 | 0 | 0 | 0 |
| 2 | 100* | 0 | 35 | 0 | 0** | 0 |
| 3 | - | 100 | 100 | 100 | 100** | 100 |
| 4 | 50* | 0 | 0 | 5 | 0** | 0 |
| 5 | 100* | 100 | 50 | 95 | 90** | 100 |
| 6 | - | 100 | 100 | 75 | 50 | 80 |
| 7 | 100 | 0 | 100 | 25 | 100 | 80 |
| 8 | 100* | 0 | 60 | 90 | 95** | 95 |
| 9 | 100* | 0 | 15 | 10 | 15** | 15 |
| 11 | 25* | .0 | 75 | 0 | 0** | 0 |
| 13 | - | 0 | 0 | 0 | 90 | 100 |
| 15 | 100* | 90 | 95 | 25 | 25 | 30 |
| 16 | 100* | 100 | 100 | 100 | 100 | 100 |
| 17 | - | 50 | 100 | 100 | 100 | 100 |
| 18 | 45 | 0 | 70 | 25 | 0 | 15· |
| 19 | 30 | 0 | 95 | ·15 | 0 | 5 |
| 20 | 100 | 90 | 100 | 100 | 100 | 100 |
| 21 | 0 | 0 | 0 | 10 | 0 | 35 |
| 22 | 100* | 100 | 100 | 60 | 100** | 100 |
| 23 | 0 | 0 | 0 | 15 | 0 | 10 |

CLAIMS:

1. A compound having the formula $RCH(SR^2)NHR^1$, wherein R is $C_1$-$C_{30}$ saturated or unsaturated aliphatic, cycloaliphatic or aromatic hydrocarbyl group or a hydrocarbyl group having one to three substituents, the substituent being halogen, hydroxy, $C_1$-$C_8$ alkoxy, $C_1$-$C_8$ alkylthio, $C_6$-$C_{10}$ aryloxy, $C_2$-$C_{10}$ dialkylamino, $C_2$-$C_{12}$ alkoxycarbonyl, methylenedioxy, nitro or cyano; or R is a 5-6 membered heterocyclic moiety containing one or more nitrogen, sulfur or oxygen atoms; $R^1$ and $R^2$ may be the same or different and are $C_1$-$C_{18}$ aliphatic or cycloaliphatic saturated or unsaturated hydrocarbyl group, provided that if R, $R^1$ or $R^2$ are unsaturated hydrocarbyl groups such unsaturation is not in the 1-position of such radials.

2. The compound of claim 1 wherein R, $R^1$ and $R^2$ have the meanings indicated for such radicals in Tables A,B, C, D, E and G of the specification.

3. A reaction mixture consisting essentially of compounds having the structural formulae $RCH=NR^1$, $R^2SH$ and $RCH(SR^2)NHR^1$, wherein R is $C_1$-$C_{30}$ saturated or unsaturated aliphatic, cycloaliphatic or aromatic hydrocarbyl group or such a hydrocarbyl group having 1 to 3 substituents, the substituents being halogen, hydroxy, $C_1$-$C_8$ alkoxy, $C_1$-$C_8$ alkylthio, $C_6$-$C_{10}$ aryloxy, $C_2$-$C_{10}$ dialkylamino, $C_2$-$C_{12}$ alkoxycarbonyl, methylenedioxy, nitro or cyano; or R is a 5-6 membered heterocyclic moiety containing one or more nitrogen, sulfur or oxygen atoms; $R^1$ and $R^2$ may be the same or different and are $C_1$-$C_{18}$ saturated or unsaturated aliphatic or cycloaliphatic hydrocarbyl group; provided that if R, $R^1$ or $R^2$ are unsaturated hydrocarbyl radicals, such unsaturation is not in the 1- position of such radicals; in an aprotic solvent inert to the compounds.

4. The mixture of claim 5 wherein R, $R^1$ and $R^2$ have the meanings indicated for such radicals in Tables A,B,C, D, E, F and G of the specification.

5. A method for preparing a compound having the formula $RCH(SR^2)NHR^1$ comprising reacting a compound having the formula $R^2SH$ and a compound having the formula $RCH=NR^1$, wherein R, $R^1$ and $R^2$ have the meanings recited in claim 1 in in an aprotic solvent inert towards the reactants at a temperature fo from -10 to 75$^{\circ}$C.

6. The method of claim 5 wherein said reaction is carried out at a temperature of from 0 to 30$^{\circ}$C.

7. The method of claim 5 wherein said reaction is carried out at room temperature.

8. The method of claim 5,6 or 7 wherein R,R$^1$ and R$^2$ have the meanings indicated for such radicals in Tables A,B,C,D, E,F and G of the specification.

9. A compound having the formula R$^2$SCH(R)N(R$^1$)C(X)NHR$^3$, wherein the radicals R,R$^1$,R$^2$,R$^3$ and X have the meanings indicated in Tables A,B,C,D,E,F and G of the specification.

10. A method for controlling weeds comprising applying to the locus of such weeds a herbicidally effective amount of a compound having the formula R$^2$SCH(R)N(R$^1$)C(X)NHR$^3$, wherein R,R$^1$,R$^2$,R$^3$ and X have the meanings indicated in Tables A,B,C,D,E,F and G of the specification; and an inert carrier therefor.

11. A herbicidal composition comprising a compound having the formula R$^2$SCH(R)N(R$^1$)C(X)NHR$^3$, wherein R,R$^1$,R$^2$, R$^3$ and X have the meanings indicated in Tables A,B,C,D,E, F and G of the specification; and an inert carrier therefor.